(19) Europäisches Patentamt
European Patent Office
Office européen des brevets

(11) Veröffentlichungsnummer: **0 577 025 A2**

# (12) EUROPÄISCHE PATENTANMELDUNG

(21) Anmeldenummer: **93110196.8**

(22) Anmeldetag: **25.06.93**

(51) Int. Cl.[5]: **A61K 31/41**, A61K 31/44, A61K 31/505

(30) Priorität: **01.07.92 DE 4221535**
**27.08.92 DE 4228555**

(43) Veröffentlichungstag der Anmeldung: **05.01.94 Patentblatt 94/01**

(84) Benannte Vertragsstaaten: **AT BE CH DE DK ES FR GB GR IE IT LI LU NL PT SE**

(71) Anmelder: **HOECHST AKTIENGESELLSCHAFT**
**D-65926 Frankfurt(DE)**

(72) Erfinder: **Heitsch, Holger, Dr.**
**Martin-Wohmann-Strasse 27**
**D-6238 Hofheim/Ts.(DE)**
Erfinder: **Wiemer, Gabriele, Prof. Dr.**
**Ernst-Moritz-Arndt-Strasse 21**
**D-6242 Kronberg/Ts.(DE)**
Erfinder: **Wagner, Adalbert, Dr.**
**Im Höhlchen 40**
**D-6234 Hattersheim/Main(DE)**
Erfinder: **Kleemann, Heinz-Werner, Dr.**
**An der hohlen Eiche 3**
**D-6380 Bad Homburg(DE)**

## (54) Angiotensin-II-Rezeptorantagonisten zur Behandlung und Prophylaxe von koronaren Herzerkrankungen.

(57) Antagonisten für Angiotensin-II-Rezeptoren des $AT_1$-Subtyps können zur Prophylaxe und Bekämpfung von koronaren Herzerkrankungen eingesetzt werden.

Darüber hinaus sind mit diesen Verbindungen, wie z. B. Azolen der Formel (I)

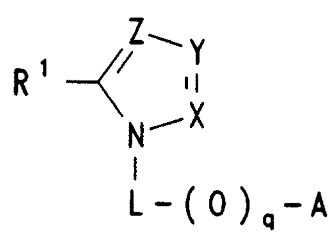

(I)

auch kognitive und erektive Dysfunktionen therapierbar.

EP 0 577 025 A2

Die vorliegende Erfindung betrifft heterocyclische Antagonisten spezieller Angiotensin-II-Rezeptoren zur Anwendung als Heilmittel für die Therapie koronarer Herzerkrankungen, wie z.B. der angina pectoris oder des septischen Schocks sowie kognitiven und erektilen Dysfunktionen sowie auch von Nieren- und Immunerkrankungen.

Den genannten Krankheiten ist gemeinsam, daß sie durch Substanzen, die analog wie z.B. das Nitroglycerin den NO-Spiegel im Organismus erhöhen, behandelt werden können. Die beschriebenen Verbindungen steigern die körpereigene NO-Bildung.

Unter anderem aus EP-A 399 731, EP-A 399 732, EP-A 400 835 und EP-A 434 038 sind Imidazolanellierte, aromatische Verbindungen als Antagonisten von Angiotensin-II-Rezeptoren bekannt.

Aus EP-A 28 834, EP-A 253 310, EP-A 401 030 und EP-A 324 377 sind Imidazolderivate und deren Verwendung als Antagonisten von Angiotensin-II-Rezeptoren bekannt.

Die vorliegende Erfindung betrifft ganz allgemein den Einsatz von Antagonisten für Angiotensin-II-Rezeptoren des $AT_1$-Subtyps zur Bekämpfung der oben genannten Krankheiten.

Diese speziellen $AT_1$-Rezeptoren werden z. B. von F. M. Bumpus et al. in Hypertension 17 (1991), Seiten 720 bis 721 beschrieben.

Insbesondere als Antagonisten für diesen Rezeptor-Subtyp geeignet sind Verbindungen der Formeln (I), (II), (III) und (IV), deren Struktur und Herstellung nachfolgend erläutert wird. Diese Verbindungen haben sich aufgrund ihrer Metabolisierung insbesondere im Menschen als vorteilhaft erwiesen.

Die Erfindung betrifft somit den Einsatz von Verbindungen der Formel (I),

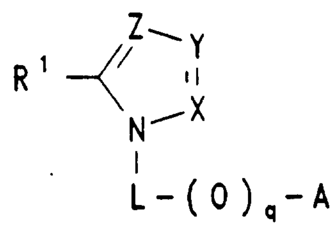

(I)

in welcher die Symbole folgenden Bedeutung haben:

a) X, Y und Z gleich oder verschieden sind und N oder $CR^2$

b) $R^1$

1. $(C_1-C_{10})$-Alkyl,
2. $(C_3-C_{10})$-Alkenyl,
3. $(C_3-C_{10})$-Alkinyl,
4. $-OR^3$
5. $(C_3-C_8)$-Cycloalkyl,
6. $(C_4-C_{10})$-Cycloalkylalkyl,
7. $(C_5-C_{10})$-Cycloalkylalkenyl,
8. $(C_5-C_{10})$-Cycloalkylalkinyl,
9. $-(CH_2)_m-B-(CH_2)_n-R^4$,
10. -Benzyl
11. einem wie unter b) 1., 2., 3. oder 9. definierten Rest, der mit $CO_2R^3$ monosubstituiert ist,
12. einem wie unter b) 1., 2., 3. oder 9. definierten Rest, worin 1 bis alle H-Atome durch Fluor substituiert sind, oder
13. den unter b) 10. definierten Rest, der am Phenyl mit 1 oder 2 gleichen oder verschiedenen Resten aus der Reihe Halogen, $(C_1-C_4)$-Alkoxy und Nitro substituiert ist,

c) $R^2$

1. Wasserstoff,
2. Halogen,
3. Nitro,
4. $C_vF_{2v+1}$
5. Pentafluorphenyl
6. Cyano,
7. $-O-R^6$
8. Phenyl
9. Phenyl-$(C_1-C_3)$-alkyl
10. $(C_1-C_{10})$-Alkyl

11. $(C_3\text{-}C_{10})$-Alkenyl
12. Phenyl-$(C_2\text{-}C_6)$-Alkenyl
13. 1-Imidazolyl-$(CH_2)_m$-,
14. 1,2,3-Triazolyl-$(CH_2)_n$-,
15. Tetrazolyl-$(CH_2)_m$-,
16. $-(CH_2)_{o-1}-CHR^7-OR^5$,
17. $-(CH_2)_o-O-CO-R^3$,
18. $-(CH_2)_o-S-R^6$,
19. $-S(O)_r-R^{19}$,
20. $-CH = CH-(CH_2)_m-CHR^3-OR^6$,
21. $-CH=CH-(CH_2)_m-CO-R^8$,
22. $-CO-R^8$,
23. $-CH=CH-(CH_2)_m-O-CO-R^7$,
24. $-(CH_2)_m-CH(CH_3)-CO-R^8$,
25. $-(CH_2)_o-CO-R^8$,
26.

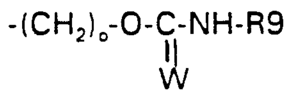

27.

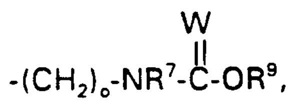

28. $-(CH_2)_o-NR^7-CO-NHR^9$,
29. $-(CH_2)_o-NR^7-SO_2R^9$,
30.

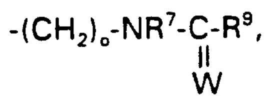

31. $-(CH_2)_nF$,
32. $-(CH_2)_n-O-NO_2$,
33. $-CH_2-N_3$,
34. $-(CH_2)_n-NO_2$,
35. $-CH=N-NR^5R^7$
36. Phthalimido-$(CH_2)_n$-,
37.

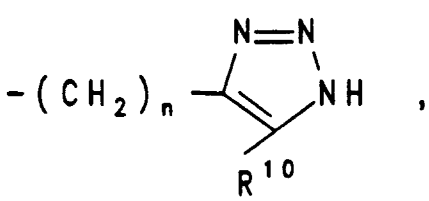

38.

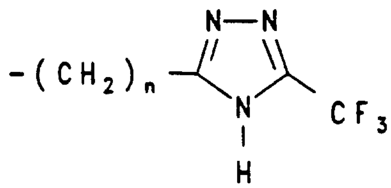
,

39.

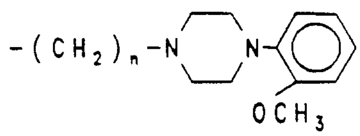
,

40.

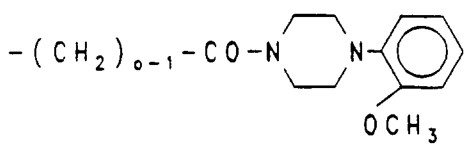
,

41. Phenyl-$SO_2$-NH-N = CH-,
42.

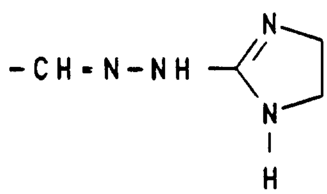
,

43. -$(CH_2)_n$-$SO_2$-$NR^7$-CS-$NR^6R^9$,
44. -$(CH_2)_nSO_2$-$NR^7$-CO-$NR^6R^9$,
45. -$(CH_2)_o$-$SO_2R^9$
46. einem wie unter c) 8. oder 9. definiertem Rest, der am Phenyl mit 1 oder 2 gleichen oder verschiedenen Resten aus der Reihe Halogen, Hydroxy, Methoxy, Trifluormethyl, $CO_2R^3$ und Phenyl substituiert ist,
47. einem wie unter c) 10., 11. oder 19. definierten Rest, worin ein bis alle H-Atome durch Fluor substituiert sind,
48. den unter c) 14. definierten Rest, der mit 1 oder 2 gleichen oder verschiedenen Resten aus der Reihe Methoxycarbonyl und ($C_1$-$C_4$)-Alkyl substituiert ist,
49. -$(CH_2)_n$-$SO_2$-$NR^7$-CO-$R^6$,
50. -$(CH_2)_n$-$SO_2$-$NR^7$CS-$R^6$,

d) $R^3$

1. Wasserstoff,
2. ($C_1$-$C_8$)-Alkyl,
3. ($C_3$-$C_8$)-Cycloalkyl,
4. Phenyl,
5. Benzyl,
6. den unter d) 2. definiertem Rest, worin 1 bis alle H-Atome durch Fluor substituiert sind;

e) $R^4$

1. Wasserstoff,

2. $(C_1-C_6)$-Alkyl,
3. $(C_3-C_8)$-Cycloalkyl,
4, $(C_2-C_4)$-Alkenyl oder
5. $(C_2-C_4)$-Alkinyl;

f) $R^5$
1. Wasserstoff,
2. $(C_1-C_6)$-Alkyl,
3. $(C_3-C_8)$-Cycloalkyl,
4. Phenyl oder
5. Benzyl;

g) $R^6$, $R^9$ gleich oder verschieden
1. Wasserstoff,
2. $(C_1-C_6)$-Alkyl, das mit 1-3 Resten aus der Reihe $(C_1-C_6)$-Alkoxy, das seinerseits mit 1-3 Resten aus der Reihe Hydroxy, $(C_1-C_6)$-Alkoxy, Amino, Mono-$(C_1-C_6)$-Alkylamino, Di-$(C_1-C_6)$-Alkylamino substituiert sein kann, $(C_2-C_{10})$-Alkenyl, Hydroxy, Amino, Mono-$(C_1-C_6)$Alkylamino, Di-$(C_1-C_6)$-Alkylamino, $(C_1-C_6)$Alkoxycarbonylamino, $(C_6-C_{12})$-Aryl-$(C_1-C_4)$Alkoxycarbonylamino; $(C_6-C_{10})$-Aryl, $(C_6-C_{10})$Aryl-$(C_1-C_3)$-Alkyl, $(C_1-C_9)$-Heteroaryl, Carboxy und $(C_1-C_4)$-Alkoxycarbonyl substituiert sein kann;
3. $(C_3-C_8)$-Cycloalkyl, wobei der Cycloalkylteil noch durch 1-3 Reste aus der Reihe $(C_1-C_4)$-Alkyl und $(C_2-C_4)$-Alkenyl substituiert sein kann;
4. $(C_3-C_8)$-Cycloalkyl-$(C_1-C_3)$-alkyl,
5. $(C_6-C_{12})$-Aryl, vorzugweise Phenyl,
6. $(C_6-C_{10})$-Aryl-$(C_1-C_4)$-Alkyl,
7. $(C_1-C_9)$-Heteroaryl, welches teilweise oder vollständig hydriert sein kann,
8. einen wie unter g) 5., 6., 7., 9., 15., 16., 17., 19., 20. oder 21. definiertem Rest, substituiert mit 1 oder 2 gleichen oder verschiedenen Resten aus der Reihe Halogen, Hydroxy, $(C_1-C_4)$-Alkyl, Methoxy, Nitro, Cyano, $CO_2R^3$, Trifluormethyl, $NR^{11}R^{12}$ und

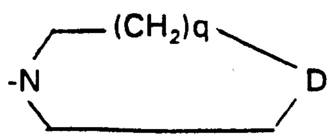

9. $(C_1-C_9)$-Heteroaryl-$(C_1-C_3)$-alkyl, wobei der Heteroarylteil teilweise oder vollständig hydriert sein kann,
10. $(C_1-C_6)$-Alkyl, worin 1 bis alle H-Atome durch Fluor substituiert sind,
11. $(C_2-C_{10})$-Alkenyl, $(C_2-C_{10})$-Alkenoyl oder $(C_2-C_{10})$-Alkadienyl,
12. $(C_3-C_8)$-Cycloalkenyl,
13. $(C_3-C_8)$-Cycloalkenyl-$(C_1-C_3)$-Alkyl,
14. Bi - oder tricyclisches $(C_4-C_{10})$-Cycloalkenyl-$(C_1-C_4)$-alkyl, das noch mit 1-3 $(C_1-C_4)$-Alkylresten substituiert sein kann;
15. $(C_6C_{10})$-Aryl-$(C_1-C_4)$-Alkyl,
16. $(C_6-C_{10})$-Aryl-$(C_3-C_6)$-Alkenyl,
17. $(C_1-C_9)$-Hetaryl-$(C_3-C_6)$-Alkenyl,
18. $(C_3-C_6)$-Alkinyl,
19. $(C_6-C_{10})$-Aryl-$(C_3-C_6)$-Alkinyl,
20. $(C_1-C_9)$-Hetaryl-$(C_3-C_6)$-Alkinyl,
21. $R^6$, $R^9$ gemeinsam mit dem sie tragenden N-Atom ein Hetaryl, das auch teilweise oder vollständig hydriert sein kann;

h) $R^7$
1. Wasserstoff,
2. $(C_1-C_6)$-Alkyl,
3. $(C_3-C_8)$-Cycloalkyl,
4. $(C_6-C_{12})$-Aryl-$(C_1-C_6)$-alkyl, vorzugsweise Benzyl,
5. Phenyl oder
6. $(C_1-C_9)$-Heteroaryl;

i) $R^8$
1. Wasserstoff,

2. $(C_1 C_6)$-Alkyl,
3. $(C_3\text{-}C_8)$-Cycloalkyl,
4. Phenyl-$(CH_2)_q$-,
5. $OR^6$,
6. $NR^{11}R^{12}$ oder
7.

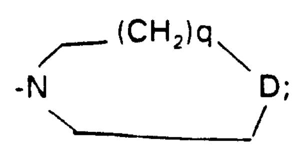

j) $R^{10}$ Cyano, Nitro oder $CO_2R^7$;
k) $R^{11}$ und $R^{12}$ gleich oder verschieden sind und
1. Wasserstoff,
2. $(C_1\text{-}C_4)$-Alkyl,
3. Phenyl,
4. Benzyl, oder
5. $\alpha$-Methylbenzyl;
l) D $NR^{13}$, O oder $CH_2$;
m) $R^{13}$ Wasserstoff, $(C_1\text{-}C_4)$Alkyl oder Phenyl;
n) A Biphenylrest, der mit bis zu 4, vorzugsweise bis zu 2 gleichen oder verschiedenen Resten $R^{14}$ oder $R^{15}$ substituiert sein kann;
o) $R^{14}$
1. Halogen,
2. Nitroso,
3. Nitro,
4. Amino,
5. Cyano,
6. Hydroxy,
7. $(C_1\text{-}C_6)$-Alkyl,
8. $(C_1\text{-}C_4)$-Alkanoyl,
9. $(C_1\text{-}C_4)$-Alkanoyloxy,
10. $CO_2R^3$,
11. Methansulfonylamino,
12. Trifluormethansulfonylamino,
13. -CO-NH-$OR^9$,
14. -$SO_2$-$NR^6R^7$,
15. -$CH_2$-$OR^7$,
16. $(C_1\text{-}C_9)$-Heteroaryl-$(CH_2)_q$-, vorzugsweise 1-Tetrazolyl,
17. $(C_7\text{-}C_{13})$-Aroyl,
18.

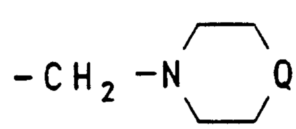

19.

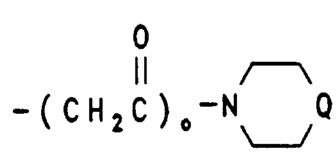

oder

20. ($C_6$-$C_{12}$)-Aryl;

p) $R^{15}$

1. Wasserstoff
2. ($C_1$-$C_6$)-Alkyl,
3. ($C_3$-$C_8$)-Cycloalkyl,
4. ($C_6$-$C_{12}$)-Aryl,
5. ($C_7$-$C_{13}$)-Aroyl,
6. ($C_1$-$C_4$)-Alkoxy,
7. ($C_1$-$C_4$)-Alkanoyloxy,
8. ($C_1$-$C_9$)-Heteroaryl,
9. $CO_2R^3$,
10. Halogen,
11. Cyano,
12. Nitro,
13. $NR^6R^7$,
14. Hydroxy,
15. $-CO-NH-CHR^5-CO_2R^3$,
16. Sulfo,
17. $-SO_3R^3$,
18. $-SO_2-NR^7-CO-NR^6R^9$ oder $-SO_2-NR^7-CS-NR^6R^9$,
19. $-NR^7-CO-NR^6-SO_2-CH_2-R^5$,
20. $-C(CF_3)_2OH$,
21. Phosphonooxy,
22. $-PO_3H_2$,
23. $-NH-PO(OH)_2$,
24. $-S(O),R^6$,
25. $-CO-R^8$,
26. $-CO-NR^6R^9$,
27. $-CR^{20}(OH)-PO(OH)_2$,
28. den unter o) 20. definiertem Rest,
29.

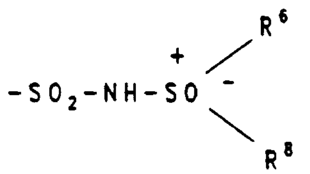

,

30.

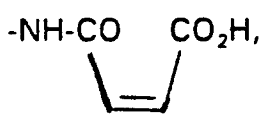

31.

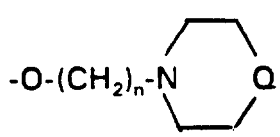

,

32. 5-Tetrazolyl-NH-CO-,
33. $-CO-NH-NH-SO_2-CF_3$,

34.

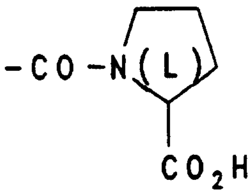

,

35.

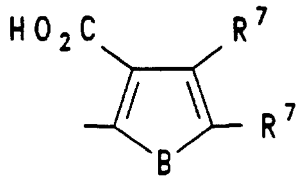

,

36.

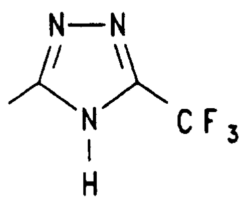

,

37.

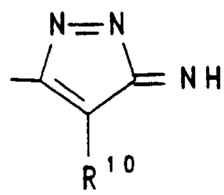

,

38.

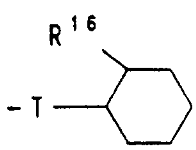

,

39.

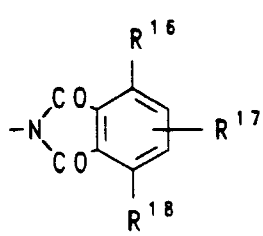

,

40. $-CO-NH-SO_2-R^{19}$
41. $-SO_2-NH-CO-R^6$ oder
42. den unter p) 4. definiertem Rest, substituiert mit 1 oder 2 gleichen oder verschiedenen Resten aus der Reihe Halogen, Cyano, Nitro, $NR^6R^7$ und Hydroxy;
43. $R^{15}$ zusammen mit $R^{14}$ bedeutet $-CO-NH-SO_2-$,
44. $-SO_2-NH-CO-O-R^6$,
45. $-SO_2-NH-SO_2-NR^6R^9$,
46. $-SO_2-NH-SO_2-R^6$,

q) B O, $NR^7$ oder S;
r) W O oder S;
s) L $(C_1-C_3)$-Alkandiyl;
t) $R^{16}$ $CO_2R^3$ oder $CH_2CO_2R^3$;
u) $R^{17}$ Wasserstoff, Halogen, $(C_1-C_4)$-Alkyl oder $(C_1-C_4)$-Alkoxy;
v) $R^{18}$ Wasserstoff, $(C_1-C_4)$-Alkyl oder Phenyl;
w) $R^{19}$

1. $(C_1-C_6)$-Alkyl,
2. $(C_3-C_8)$-Cycloalkyl,
3. Phenyl
4. Benzyl oder
5. den unter w) 1. definiertem Rest, worin ein bis alle H-Atome durch Fluor substituiert sind.

x) T

1. eine Einfachbindung,
2. -CO-,
3. $-CH_2-$,
4. -O-,
5. -S-,
6. $-NR^{21}-$,
7. $-CO-NR^{21}$,
8. $NR^{21}-CO-$,
9. $-O-CH_2-$,
10. $-CH_2-O-$,
11. $-S-CH_2-$,
12. $-CH_2-S$,
13. $-NH-CR^{20}R^{22}$,
14. $-NR^{21}-SO_2$,
15. $SO_2-NR^{21}-$,
16. $-CR^{20}R^{22}-NH$,
17. $-CH=CH-$,
18. $-CF=CF-$,
19. $-CH=CF-$,
20. $-CF=CH-$,
21. $-CH_2-CH_2-$,
22. $-CF_2-CF_2-$,
23. $-CH(OR^3)-$,
24. $-CH(OCOR^5)-$,
25.

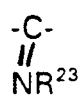

oder
26.

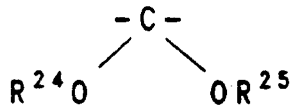 ;

y) $R^{20}$ und $R^{22}$ gleich oder verschieden
Wasserstoff, $(C_1\text{-}C_5)$-Alkyl, Phenyl, Allyl oder Benzyl;
z) $R^{21}$ Wassserstoff, $(C_1\text{-}C_6)$Alkyl, Benzyl oder Allyl;
a') $R^{23}$
1. $NR^{20}R^{21}$
2. Ureido,
3. Thioureido,
4. Toluol-4-sulfonyl oder
5. Benzolsulfonyoamino

b') $R^{24}$ und $R^{25}$ gleich oder verschieden $(C_1\text{-}C_4)$-Alkyl oder gemeinsam $-(CH_2)_q-$,
c') Q $CH_2$, NH, O oder S;
d') m eine ganze Zahl von 0 bis 5;
e') n eine ganze Zahl von 1 bis 5;
f') o eine ganze Zahl von 1 bis 10;
g') q 0 oder 1;
h') r 0, 1 oder 2 ist, oder
i') v eine ganze Zahl von 1 bis 6;

sowie deren physiologisch verträglichen Salze.

Alkyl, Alkenyl und Alkinyl können geradkettig oder verzweigt sein. Entsprechendes gilt für davon abgeleitete Reste, wie Alkanoyl oder Alkoxy. Unter Cycloalkyl werden auch alkylsubstituierte Ringe verstanden. $(C_6\text{-}C_{12})$-Aryl ist beispielsweise Phenyl, Naphthyl oder Biphenylyl vorzugsweise Phenyl. Entsprechendes gilt für davon abgeleitete Reste, wie Aroyl und Aralky.

Unter $(C_1\text{-}C_9)$-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pryrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxylinyl, Chinazolinyl, Cinnolinyl.

Gegebenenfalls vorkommende Stereozentren können sowohl (R)- als auch (S)-konfiguriert sein.

Die Verknüpfung von A erfolgt über eine Alkandiyl-Brücke L, welche vorzugsweise eine Methylengruppe ist. Die Methylengruppe ist vorzugsweise direkt an den Biphenylrest gebunden.

Unter physiologisch verträglichen Salzen von Verbindungen der Formel (I) versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basiche Gruppen sind unter anderen Salze der Salzsäure, Schwefelsäure, Phosphorsäure oder von Carbonsäuren oder Sulfonsäuren, wie z. B. Essigsäure, Zitronensäure, Benzoesäure, Maleinsäure, Fumarsäure, Weinsäure und p-Toluolsulfonsäure bevorzugt.

Bevorzugt werden ferner bei der Behandlung und Prophylaxe der genannten Erkrankungen Verbindungen der Formel (I) eingesetzt, worin
X N, Y $CR^2$ und Z $CR^2$;
X $CR^2$, Y N und Z $CR^2$;
X $CR^2$, Y $CR^2$ und Z N oder
X, Y und Z jeweils N,
a) $R^1$
1. $(C_1\text{-}C_{10})$-Alkyl,
2. $(C_3\text{-}C_{10})$-Alkenyl,
3. $(C_3\text{-}C_{10})$-Alkinyl,
4. $(C_3\text{-}C_8)$-Cycloalkyl,
5. Benzyl oder

6. Benzyl, das wie oben beschrieben (b 13.) substituiert ist;

b) $R^2$

1. Wasserstoff,
2. Halogen,
3. Nitro,
4. $C_vF_{2v+1}$,
5. Pentafluorphenyl,
6. Cyano,
7. $-O-R^6$,
8. Phenyl,
9. Phenyl-($C_1$-$C_3$)-alkyl,
10. ($C_1$-$C_{10}$)-Alkyl,
11. ($C_3$-$C_{10}$)-Alkenyl,
12. Phenyl-($C_2$-$C_6$)-alkenyl,
13. 1-Imidazolyl-$(CH_2)_m$-,
14. 1,2,3-Triazolyl-$(CH_2)_o$-,
15. Tetrazolyl-$(CH_2)_m$-,
16. $-(CH_2)_o$-1-$CHR^7$-$OR^5$,
17. $-(CH_2)_o$-O-$COR^3$,
18. $-COR^8$,
19. $-(CH_2)_o$-CO-$R^8$,
20. $-S(O)_rR^{19}$,
21. -CH = CH-$(CH_2)_m$-$CHR^{3-}OR^6$,
22. -CH = CH-$(CH_2)_m$-CO-$R^8$,
23. $-(CH_2)_o$-NH-CO-$OR^9$,
24. $-(CH_2)_o$-NH-$SO_2$-$R^9$,
25. $-(CH_2)_nF$,
26. $-(CH_2)_o$-$SO_3R^9$,
27. $-(CH_2)_n$-$SO_2$-NH-CO-$NR^6R^9$,
28. $-(CH_2)_n$-$SO_2$-NH-CS-$NR^6R^9$, oder
29. einem wie unter b) 8., 9., 10., 11. oder 14. definierten Rest, der wie oben unter c) 46., 47. oder 48. jeweils wie für einen solchen Rest beschrieben substituiert ist,
30. $-(CH_2)_n$-$SO_2$-$NR^7CO$-$R^8$,
31. $-(CH_2)_n$-$SO_2$-$NR^7$-CS-$R^6$;

c) $R^8$ Wasserstoff; ($C_1$-$C_5$)-Alkyl, $OR^6$, $NR^{11}R^{12}$ oder Morpholino;

d) T

1. eine Einfachbindung,
2. -CO-,
3. $-CONR^{21}$-,
4. $-CH_2$-$CH_2$-,
5. $-NR^{21}$-CO-,
6. -O-$CH_2$-,
7. $-CH_2$-O-,
8. -S-$CH_2$-,
9. $-CH_2$-S-,
10. -NH-$CH_2$-,
11. $-CH_2$-NH- oder 12. -CH = CH-und die übrigen Reste und Variablen wie oben definiert sind.

Besonders bevorzugt sind Verbindungen der Formel (I), wobei bedeuten:

X N, Y $CR^2$ und Z $CR^2$;
X $CR^2$, Y N und Z $CR^2$;
X $CR^2$, Y $CR^2$ und Z N oder
X, Y und Z jeweils N,

a) $R^1$ ($C_1$-$C_7$)-Alkyl, ($C_3$-$C_{10}$)-Alkenyl oder ($C_3$-$C_7$)-Alkinyl;

b) $R^2$

1. Chlor,
2. Brom,
3. $C_vF_{2v+1}$ mit v = 1, 2 oder 3,

4. Pentafluorphenyl,
5. O-$R^6$,
6. -$S(O)_rR^{19}$,
7. $(CH_2)_o$-1-$CHR^7$-$OR^5$,
8. $(CH_2)_o$-O-CO-$R^3$,
9. -$COR^8$,
10. -$(CH_2)_o$-CO-$R^8$,
11. -$CH_2$-NH-CO-$R^8$,
12. -$(CH_2)_o$-NH-$SO_2$-$R^9$,
13. -CH = CH-$CHR^3$-$OR^6$,
14. Tetrazolyl-$(CH_2)_m$-,
15. -$(CH_2)_nSO_2$-NH-CO-$NR^6R^9$,
16. -$(CH_2)_o$-$SO_3R^9$ oder

gegebenenfalls durch Hydroxy substituiertes ($C_1$-$C_6$)-Alkyl, vorzugsweise Hydroxymethyl;

c) $R^3$ Wasserstoff, ($C_1$-$C_4$)-Alkyl oder Benzyl;

d) $R^6$, $R^9$ gleich oder verschieden

1. Wasserstoff,
2. ($C_1$-$C_6$)-Alkyl, das mit 1 bis 3 Resten aus der Reihe ($C_1$-$C_6$)-Alkoxy, das seinerseits mit 1 bis 3 Resten aus der Reihe Hydroxy, ($C_1$-$C_6$)-Alkoxy, Amino, Mono-($C_1$-$C_6$)-Alkylamino, Di-($C_1$-$C_6$)-Alkylamino substituiert sein kann, ($C_2$-$C_{10}$)-Alkenyl, Hydroxy, Amino, Mono-($C_1$-$C_6$)Alkylamono, Di-($C_1$-$C_6$)-Alkylamino, ($C_1$-$C_6$)Alkoxycarbonylamino, ($C_6$-$C_{12}$)-Aryl-($C_1$-$C_4$)Alkoxycarbonylamino; ($C_6$-$C_{10}$)-Aryl, ($C_6$-$C_{10}$)Aryl-($C_1$-$C_3$)-Alkyl, ($C_1$-$C_9$)-Heteroaryl, Carboxy und ($C_1$-$C_4$)-Alkoxycarbonyl substituiert sein kann;
3. ($C_3$-$C_6$)-Cycloalkyl,
4. ($C_3$-$C_6$)-Cycloalkyl-($C_1$-$C_3$)-alkyl,
5. Phenyl,
6. Phenyl-($C_1$-$C_3$)-Alkyl,
7. ($C_1$-$C_7$)-Heteroaryl, welches teilweise oder vollständig hydriert sein kann,
8. einen wie oben unter g) 5., 6., 7. oder 9., 14. bis 16. und 18. bis 20. definierten Rest, substituiert mit 1 oder 2 gleichen oder verschiedenen Resten aus der Reihe Halogen, Hydroxy, ($C_1$-$C_4$)-Alkyl, Methoxy, Nitro, Cyano,

$CO_2R^3$, Trifluormethyl, -$NR^{11}R^{12}$ und

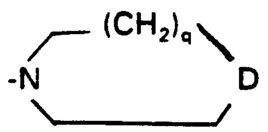 ,

9. ($C_1$-$C_9$)-Heteroaryl-($C_1$-$C_3$)-alkyl, wobei der Heteroarylteil teilweise oder vollständig hydriert sein kann,
10. ($C_1$-$C_6$)-Alkyl, worin 1 bis alle H-Atome durch Fluor substituiert sind,
11. ($C_2$-$C_4$)-Alkenyl oder ($C_3$)-Alkenoyl,
12. ($C_3$-$C_6$)-Cycloalkenyl,
13. ($C_3$-$C_6$)-Cycloalkenyl-($C_1$-$C_3$)-Alkyl,
14. Bi- oder tricyclisches ($C_4$-$C_{10}$)-Cycloalkenyl-($C_1$-$C_{10}$)-alkyl, das noch mit 1 bis 3 ($C_1$-$C_4$)-Alkylresten substituiert sein kann;
15. ($C_6$-$C_{10}$)-Aryl-($C_1$-$C_3$)-Alkyl,
16. ($C_6$-$C_{10}$)-Aryl-($C_3$-$C_6$)-Alkenyl,
17. ($C_1$-$C_6$)-Hetaryl-($C_3$-$C_6$)-Alkenyl,
18. ($C_3$-$C_6$)-Alkinyl,
19. ($C_6C_{10}$)-Aryl-($C_3$-$C_6$)-Alkinyl,
20. ($C_1$-$C_6$)-Hetaryl-($C_3$-$C_6$)-Alkinyl,
21. $R^6$, $R^9$ gemeinsam mit dem sie tragenden N-Atom ein Hetaryl, das auch teilweise oder vollständig hydriert sein kann;

e) $R^7$ Wasserstoff, ($C_1$-$C_4$)-Alkyl, ($C_1$-$C_9$)-Heteroaryl, oder ($C_6$-$C_{12}$)-Aryl-($C_1$-$C_4$)-Alkyl;

f) $R^{14}$

1. ($C_1$-$C_4$)-Alkyl,

2. $(C_1-C_4)$-Alkoxy,
3. Cyano,
4. Amino,
5. Nitroso,
6. Nitro,
7. Fluor,
8. Chlor,
9. Brom,
10. $(C_1-C_9)$-Heteroaryl-$CH_2$-,
11. $(C_1-C_4)$-Alkanoyloxy,
12. $(C_1-C_4)$Alkanoyl,
13. Benzoyl,
14. -NH-CO-$R^7$ oder
15. Tetrazolyl;

h) $R^{15}$

1. $(C_1-C_4)$-Alkyl,
2. $(C_6-C_{12})$-Aryl,
3. $(C_1-C_3)$-Alkanoyloxy,
4. $(C_1-C_4)$-Alkoxy,
5. $(C_1-C_9)$-Heteroaryl, vorzugsweise 5-Tetrazolyl,
6. Cyano,
7. Nitro,
8. Hydroxy,
9. -S(O),$R^6$,
10. -$SO_3R^3$,
11. Chlor,
12. Brom,
13. Benzoyl,
14. -$CO_2R^3$,
15. -CO-NH-$R^6$,
16. -CO-$R^8$,
17. -$SO_2$-$NR^6R^7$,
18. -$SO_2$-NH-CO-$NR^6R^9$,
19. -$PO_3H_2$,
20. -CO-$CHR^5$-$CO_2H$,
21. -NH-CO-NH-$SO_2$-$CH_2$-$R^5$,
22. 5-Tetrazolyl-NH-CO-,
23.

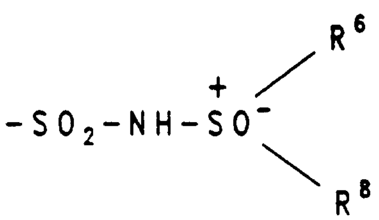

,

24.

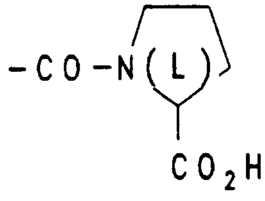

,

25.

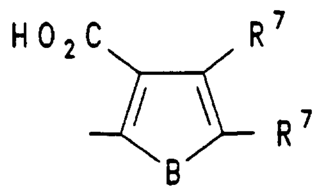 ,

26.

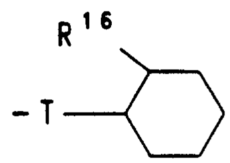 ,

27.

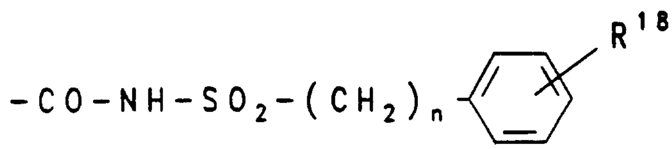

oder

28. den unter h) 2) definierten Rest, substituiert wie oben definiert (siehe p) 42),

29. $R^{15}$ mit $R^{14}$ zusammen $-CO-NH-SO_2-$,

30. $-SO_2-NH-COO-R^6-$

31. $-SO_2-NH-SO_2-NR^6R^9$,

32. $-SO_2-NH-SO_2-R^6$;

i) $R^{18}$ Wasserstoff, Methyl oder Ethyl;

j) T eine Einfachbindung, -O-,-CO-, -NHCO- oder $-OCH_2-$;

k) q = O und L = Methylen,

und die übrigen Reste und Variablen wie oben definiert sind.

Bevorzugt sind darüberhinaus Azol-Derivate der allgemeinen Formel (I), in welcher Z ein Stickstoffatom und Y und X unabhängig voneinander $CR^2$ bedeuten, und die übrigen Symbole wie oben definiert sind.

Insbesondere geeignet sind ferner Azol-Derivate der allgemeinen Formel (I), wobei die Symbole folgende Bedeutung haben:

Z Stickstoff,

X, Y unabhängig voneinander $CR^2$,

$R^1$ $(C_1-C_7)$-Alkyl, $(C_3-C_7)$-Alkenyl oder $(C_3-C_7)$-Alkinyl,

$R^2$ Wasserstoff, Halogen, Nitro, $(C_1-C_3)$-Perfluoralkyl, Cyano, $(C_1-C_{10})$-Alkyl, $(C_3-C_{10})$-Alkenyl, $-CH_2OR^5$, $-S(O)_r-R^{19}$, $-CO-R^8$ oder $-O-R^6$,

$R^5$ Wasserstoff oder $(C_1-C_6)$-Alkyl

$R^6$, $R^9$

1. Wasserstoff,

2. $(C_1-C_6)$-Alkyl, das mit 1 bis 3 Resten aus der Reihe $(C_1-C_6)$-Alkoxy, das seinerseits mit 1 bis 3 Resten aus der Reihe Hydroxy, $(C_1-C_6)$-Alkoxy, Amino, Mono-$(C_1-C_6)$-Alkylamino, Di-$(C_1-C_6)$-Alkylamino substituiert sein kann, $(C_2-C_{10})$-Alkenyl, Hydroxy, Amino, Mono-$(C_1-C_6)$-Alkylamono, Di-$(C_1-C_6)$-Alkylamino, $(C_1-C_6)$-Alkoxycarbonylamino, $(C_6-C_{10})$-Aryl, $(C_6-C_{10})$Aryl-$(C_1-C_3)$-Alkyl, $(C_1-C_9)$-Heteroaryl, Carboxy und $(C_1-C_4)$-Alkoxycarbonyl substituiert sein kann;

3. $(C_3-C_8)$-Cycloalkyl,

4. $(C_3-C_6)$-Cycloalkyl-$(C_1-C_4)$-alkyl,

5. ($C_6$-$C_{12}$)-Aryl, vorzugsweise Phenyl,
6. ($C_6$-$C_{10}$)-Aryl-($C_1$-$C_4$)-Alkyl,
7. ($C_1$-$C_9$)-Heteroaryl, welches teilweise oder vollständig hydriert sein kann,
8. ($C_1$-$C_9$)-Heteroaryl-($C_1$-$C_3$)-alkyl, wobei der Heteroarylteil teilweise oder vollständig hydriert sein kann,
9. einen wie vorstehend unter 5., 6., 7. und 8. definierten Rest, substituiert mit 1 oder 2 gleichen oder verschiedenen Resten aus der Reihe Halogen, Hydroxy, ($C_1$-$C_4$)-Alkyl, Methoxy, Nitro, Cyano, $CO_2R^3$, Trifluormethyl, $-NR^{11}R^{12}$ und

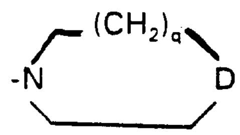

10. ($C_1$-$C_6$)-Alkyl, worin 1 bis alle H-Atome durch Fluor substituiert sind,
11. ($C_2$-$C_6$)-Alkenyl oder ($C_3$-$C_6$)-Alkenoyl,
12. ($C_3$-$C_8$)-Cycloalkenyl,
13. ($C_3$-$C_8$)-Cycloalkenyl-($C_1$-$C_3$)-Alkyl,
14. ($C_6$-$C_{10}$)-Aryl-($C_1$-$C_4$)-Alkyl,
15. ($C_6$-$C_{10}$)-Aryl-($C_3$-$C_6$)-Alkenyl,
16. ($C_1$-$C_9$)-Hetaryl-($C_3$-$C_6$)-Alkenyl,
17. ($C_3$-$C_6$)-Alkinyl,
18. ($C_6$-$C_{10}$)-Aryl-($C_3$-$C_6$)-Alkinyl 19. ($C_1$-$C_9$)-Hetaryl-($C_3$-$C_6$)-Alkinyl,
20. $R^6$, $R^9$ gemeinsam mit dem sie tragenden N-Atom ein Hetaryl, das auch teilweise oder vollständig hydriert sein kann,

| | |
|---|---|
| $R^7$ | Wasserstoff, |
| $R^8$ | Wasserstoff oder $-OR^6$, |
| $R^{11}$, $R^{12}$ | unabhängig voneinander Wasserstoff oder ($C_1$-$C_4$)-Alkyl |
| D | $-NR^{13}$, -O oder $-CH_2$, |
| $R^{13}$ | Wasserstoff oder ($C_1$-$C_4$)-Alkyl, |
| A | Biphenylrest der mit einem Rest $R^{15}$ oder mit $R^{14}$ und $R^{15}$ zusammen substituiert ist, |
| $R^{15}$ | $-SO_2-NR^7-CO-NR^6R^9$, $-SO_2-NH-COO-R^6$, $-SO_2-NH-SO_2-NR^6-R^9$, $-SO_2-NH-CO-R^6$ oder $-SO_2-NH-SO_2-R^6$; oder |
| $R^{14}$ und $R^{15}$ | zusammen $-CO-NH-SO_2-$ sein kann, |
| L | $-CH_2-$, |
| q | Null und |
| r | Null, 1 oder 2, |

sowie deren physiologisch verträgliche Salze.

Verbindungen der Formel (I), sowie deren physiologisch verträgliche Salze können dadurch hergestellt werden, daß man Verbindungen der Formel (IIa)

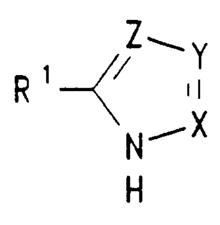

(IIa)

worin $R^1$, X, Y und Z wie oben definiert sind, alkyliert mit Verbindungen der Formel (IIIa)

$U-L-(O)_q-A$ (IIIa)

worin L, A und q wie oben definiert sind, und U für eine Fluchtgruppe steht, gegebenenfalls temporär eingeführte Schutzgruppen wieder abspaltet, erhaltene Sulfonamide der Formel (I) gegebenenfalls in Urethane der Formel (I) überführt, erhaltene Sulfonamide der Formel (I) oder erhaltene Urethane der Formel (I) überführt und die erhaltenen Verbindungen der Formel (I) gegebenenfalls in ihre physiologisch verträglichen Salze überführt.

Geeignete Fluchtgruppen U sind vorzugsweise nucleofuge Gruppen (vgl. Angew. Chem. 72 [1960] 71) wie Halogen, o-Toluolsulfonat, Mesylat oder Triflat.

Verfahren zur Herstellung der Vorstufen der Formel (IIa) sind u. a. bekannt aus US 4 355 044, EP-A-324 377 und EP-A-323 841.

Weitere Verfahren werden beschrieben von G. L'abbe (Chem. Rev. 69, 345 [1969]), T. Srodsky ("The Chemistry of the Azido Group", Wiley, New York, 1971, S. 331), H. Wamhoff ("Comprehensive Heterocyclic Chemistry) sowie von S. Katritzky Ed., Pergamon Press, New York [1984]).

Ein weiteres Verfahren zur Herstellung von Verbindungen der Formel (IIa) geht von 1-Cyanoglyoxylsäure-2-oxim-Derivaten aus und liefert nach Reduktion des Oxims mit literaturbekannten Reduktionsmitteln und Addition von Mercaptoverbindungen an die Nitrilgruppe unter Verwendung geeigneter Schutzgruppen Vorstufen, die unter Wasser-abspaltenden Bedingungen zu Imidazolen cyclisiert werden können. Für den Cyclierungsschritt können u.a. Gemische aus $PCl_5$ und Dimethylaminopyridin (DMAP), $POCl_3$ und $SOCl_2$ und deren Gemische mit DMAP verwendet werden.

Die Oxidation der Thioverbindungen der Formel (I) mit $R^2$ gleich $-S(O)_rR^{19}$, wobei r gleich Null bzw. 1 ist, zu den entsprechenden Sulfonen und Sulfoxiden erfolgt bevorzugt durch Persäuren in geeigneten Lösungsmitteln wie z. B. Dichlormethan.

Zur Alkylierung der Azole der Formel (IIa) sind z. B. entspechende Benzylhalogenide, -tosylate, -mesylate oder Triflate oder entsprechende Alkylhalogenide, -tosylate, mesylate oder -triflate geeignet.

Die Alkylierung erfolgt nach prinzipiell bekannten Verfahren in analoger Weise.

Azol-Derivat der Formel (IIa) werden z. b. in Gegenwart einer Base metalliert. Bevorzugte Basen sind Metallhydride der Formel MH wie z. B. Lithium-, Natrium- oder Kaliumhydrid in beispielsweise DMF oder DMSO als Lösungsmittel oder Metallalkoxide der Formel MOR, wobei R für Methyl, Ethyl, t-Butyl steht, und die Reaktion in dem entsprechenden Alkohol, DMF oder DMSO durchgeführt wird. Die so gebildeten Salze der Azole werden in einem aprotischen Lösungsmittel wie DMF oder DMSO gelöst und mit einer geeigneten Menge Alkylierunsreagenz versetzt.

Eine alternative Möglichkeit zur Deprotonierung der Azol-Derivate stellt z. B. die Umsetzung mit Kaliumcarbonat in DMF oder DMSO dar.

Die Umsetzungen werden bei Temperaturen unterhalb Raumtemperatur bis zum Siedepunkt des Reaktionsgemisches, vorzugsweise zwischen + 20 °C und dem Siedepunkt des Reaktionsgemisches während etwa 1 bis 10 Stunden durchgeführt.

Die Biphenylderivate können z. B. ausgehend von Arylboronsäurederivaten durch Kopplung mit substituierten Arylhalogeniden mt Übergangsmetallkatalysatoren, insbesondere Palladium synthetisiert werden. Entsprechende Reaktionen werden von R. B. Miller et al. (Organometallics 1984, 3, 1261) oder von A. Zuzuki et al. (Synthetic Commun. 11 (7), 513 [1981]) beschrieben.

Die Sulfonylurethane der Formel (I) können aus entsprechenden Sulfonamiden der Formel (I) durch Umsetzung mit Chlorkohlensäureestern in inerten hochsiedenden Lösungsmitteln wie z. B. Toluol bei Temperaturen von ca. 100 °C bzw. den Siedepunkten der entsprechenden Lösungsmitteln erhalten werden.

Analog können Sulfonyl-sulfonamide aus den entspechenden Sulfonamiden durch Umsetzung mit Sulfonsäurechloriden oder Sulfamoylchloriden hergestellt werden.

Der Sulfonamid-Rest kann, falls erforderlich, ausgehend von einer Aminogruppe mittels Meerwein-Reaktion hergestellt werden. Hierfür wird das Hydrochlorid des Amins zuerst diazotiert und dann in Gegenwart eines Kupferkatalysators mit Schwefeldioxid in Eisessig umgesetzt. Nachfolgende Einwirkung von Ammoniak führt zur Sulfonamidogruppe.

Alternativ kann ein entsprechendes Thiophenol durch Oxidation mit Chlor und anschließender Einwirkung von Ammoniak in ein Sulfonamid umgewandelt werden.

Desweiteren werden zur Behandlung von Herzrhythmusstörungen bevorzugt Verbindungen der Formel (III) eingesetzt, in welcher die Symbole folgende Bedeutung haben:

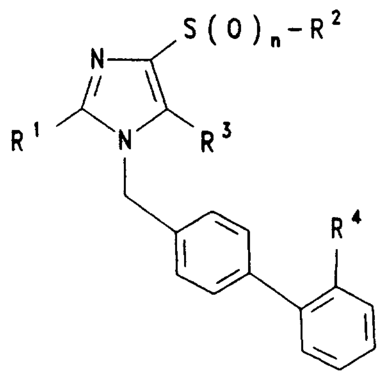

(III)

a) $R^1$ $(C_1\text{-}C_7)$-Alkyl, $(C_3\text{-}C_7)$-Alkenyl oder $(C_3\text{-}C_7)$-Alkinyl; insbesondere $(C_1\text{-}C_3)$-Alkyl, vorzugsweise n-Propyl oder Ethyl, insbesondere jedoch n-Propyl

b) $R^2$ $(C_1\text{-}C_6)$-Alkyl, vorzugsweise Methyl

c) $R^3$ $-CO-R^6$

d) $R^4$ $SO_2-NH-CO-NR^7R^9$,
$SO_2-NH-COO-R^7$ oder
$SO_2-NH-CO-R^7$,
$SO_2-N=CH-N(CH_3)_2$

e) $R^6$ Wasserstoff oder $OR^7$,

f) $R^7$, $R^9$ gleich oder verschieden Wasserstoff, $(C_1\text{-}C_6)$-Alkyl, vorzugsweise Methyl, Ethyl oder Propyl, $(C_3\text{-}C_8)$-Cycloalkyl, $(C_3\text{-}C_6)$-Cycloalkyl-$(C_1\text{-}C_3)$-alkyl, $(C_6\text{-}C_{12})$-Aryl, vorzugsweise Phenyl oder $(C_6\text{-}C_{10})$-Aryl-$(C_1\text{-}C_4)$-alkyl, $(C_2\text{-}C_6)$-Alkenyl, $(C_3\text{-}C_6)$Alkenoyl oder $(C_3\text{-}C_6)$-Alkinyl

g) n O,1 oder 2, insbesondere O;

sowie deren verträgliche Salze, da diese Verbindungen aufgrund ihrer Metabolisierung im menschlichen Körper besonders als Therapeutika geeignet sind.

Die Erfindung betrifft ferner den Einsatz von Verbindungen der Formel (II)

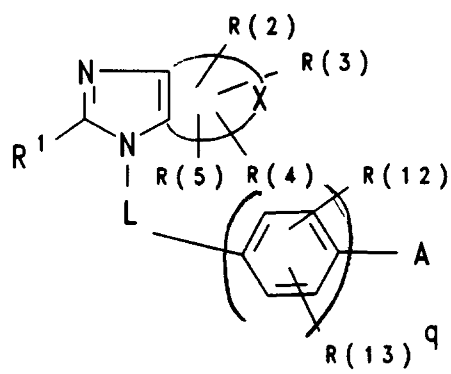

(II)

in welcher die Symbole folgende Bedeutung haben:

X steht für einen monocylischen Rest mit 3, 4 oder 5 Ringatomen oder einen bicyclischen Rest mit 8 - 10 Ringatomen, der ganz oder teilweise hydriert sein kann und in dem eine oder mehrere CH- bzw. $CH_2$-Gruppen durch N, NH oder O ersetzt sein können;

R(1)

1. $(C_1\text{-}C_{10})$-Alkyl,
2. $(C_3\text{-}C_{10})$-Alkenyl,
3. $(C_3\text{-}C_{10})$-Alkinyl,
4. OR(6),
5. $(C_3\text{-}C_8)$-Cycloalkyl,
6. $(C_4\text{-}C_{10})$-Cycloalkylalkyl,
7. $(C_5\text{-}C_{10})$-Cycloalkylalkenyl,

8. $(C_5-C_{10})$-Cycloalkinyl,
9. $(CH_2)_m$-B-$(CH_2)_n$-R(7),
10. Benzyl,
11. einem wie unter 1., 2., 3. oder 9. definiertem Rest, der monosubstituiert ist mit $CO_2R(6)$,
12. einem wie unter 1., 2., 3. oder 9. definiertem Rest, worin 1 bis H-Atome durch Fluor substituiert sind, oder
13. den unter 10. definiertem Rest, der am Phenyl mit 1 oder 2 gleichen oder verschiedenen Resten aus der Reihe Halogen, $(C_1-C_4)$-Alkoxy und Nitro substituiert ist;

R(2), R(3), R(4) und R(5) gleich oder verschieden sind und
1. Wasserstoff, Halogen, Hydroxy, Cyano, Nitro, Sulfo, Formyl, Benzoyl, $(C_1-C_6)$-Acyl, $(C_1-C_8)$-Acyloxy, Mercapto, Carboxy, $(C_1-C_4)$-Alkoxycarbonyl,
2. einen linearen oder verzweigten, gegebenenfalls substituierten, bis zu 6 C-Atome enthaltene Alkyl-, Alkenyl-,Alkoxy- oder Allylthio-Rest,
3. einen Aryl-, Arylalkyl oder Arylalkenyl-Rest, in denen der Alkyl- und Alkenyl-Substituent unverzweigt oder verzweigt bis zu 6 C-Atomen aufweist und der Aryl-Substituent steht für einen monocyclischen Rest mit 5 oder 6 Ringatomen oder für kondensierte Ringe mit 8 bis 14 Ringatomen, in denen ein oder mehrere Heteroatome wie O, N oder S enthalten sind und die gegebenenfalls substituiert sind,
4. einen Rest

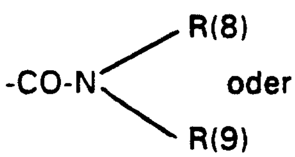 oder 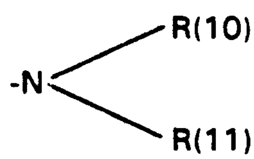

bedeuten;

R(6)
1. Wasserstoff,
2. $(C_1-C_8)$-Alkyl,
3. $(C_3-C_8)$-Cycloalkyl,
4. Phenyl,
5. Benzyl oder
6. den unter 2. definiertem Rest, worin 1 bis alle H-Atome durch Fluor substituiert sind;

R(7)
1. Wasserstoff,
2. $(C_1-C_8)$-Alkyl,
3. $(C_3-C_8)$-Cycloalkyl,
4. $(C_2-C_4)$-Alkenyl oder
5. $(C_2-C_4)$-Alkinyl,

R(8) und R(9) oder R(10) und R(11) entweder gleich oder verschieden für
1. Wasserstoff,
2. $(C_1-C_6)$-Alkyl oder $(C_1-C_6)$-Alkenyl, unsubstituiert oder substituiert druch Halogen, Hydroxy oder $(C_1-C_6)$-Alkoxy,
3. Aryl oder $(C_1-C_6)$-Alkylaryl, worin der Arylrest monocyclisch mit 5 oder 6 Ringatomeen oder bicyclisch mit 8 - 10 Ringatomen ist, gegebenenfalls ein oder mehrere Heteroatome wie O, N und S enthält und mit 1 oder 2 gleichen oder verschiedenen Resten aus der Reihe Halogen, Hydroxy, Nitro, $(C_1-C_6)$-Alkyl,

$(C_1-C_6)$-Alkenyl, $(C_1-C_4)$Alkanoyl, $(C_1-C_4)$-Alkanoyloxy und $CO_2R(6)$ substituiert ist;

oder

R(8) und R(9) und R(11) zusammen mit dem sie tragenden N-Atom einen 4-bis 8-gliedrigen Ring bilden, der gesättigt oder ungesättigt ist, ein weiteres Heteroatom ausgewählt aus der Gruppe N, O und S enthalten kann und unsubstituiert oder durch Halogen, Hydroxy-, $(C_1-C_4)$-Alkyl, $(C_1-C_4)$-Alkenyl, $(C_1-C_4)$-Alkyloxy und $CO_2R(6)$ substituiert ist.

oder

R(10) und R(11) entweder gleich oder verschieden stehen für einen Acylrest aus bis zu 6 C-Atomen oder einen $(C_1-C_6)$-Alkyl oder $(C_6-C_{12})$-Arylrest, die gegebenenfalls durch Halogen oder $(C_1-C_6)$-Alkylreste substituiert sind;

L $(C_1-C_3)$-Alkandiyl bedeutet, vorzugsweise Methylen;

R(12) und R(13) gleich oder verschieden sind und

1. Wasserstoff,
2. Halogen,
3. Nitro,
4. $(C_1-C_4)$-Alkyl oder
5. $(C_1-C_2)$-Alkoxy,

bedeuten;

q Null oder 1 ist,

A steht für entweder

1. den Rest eines Heterocyclus mit 5 - 10 Ringatomen, der mono- oder bicyclisch sein kann, und wovon bis zu 9 Ringatome C-Atome sind, der unsubstituiert oder mit bis zu 6, vorzugsweise bis zu 3 gleichen oder verschiedenen Resten R(14) und R(15) substituiert ist,

oder

2. einen Biphenylrest, der unsubstituiert oder mit bis zu 4, vorzugsweise bis zu 2 gleichen oder verschiedenen Resten R(14) und R(15) substituiert ist, wobei A aber zwingend mit mindestens einem unter R(15) 18., 19., 28., 40. oder 42. definierten Rest substituiert und q = Null ist.

R(14)

1. Halogen,
2. Oxo,
3. Nitroso,
4. Nitro,
5. Amino,
6. Cyano,
7. Hydroxy,
8. $(C_1-C_6)$-Alkyl,
9. $(C_1-C_4)$-Alkanoyl,
10. $(C_1-C_4)$-Alkanoyloxy,
11. $CO_2R(6)$,
12. Methansulfonylamino,
13. Trifluormethansulfonylamino,
14. -CO-NH-OR(16),
15. $-SO_2-NR(17)R(18)$,
16. $-CH_2-OR(18)$,
17. $(C_1-C_4)$-Heteroaryl-$(CH_2)_q$-, vorzugsweise 1-Tetrazolyl,
18. $(C_7-C_{13})$-Aroyl,
19.

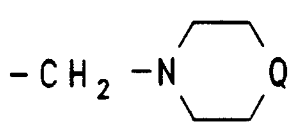

20.

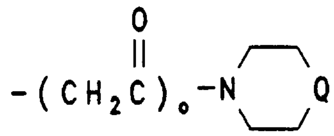

oder

21. ($C_6$-$C_{12}$)-Aryl

bedeutet;

R(15)

1. Wasserstoff,
2. ($C_1$-$C_6$)-Alkyl,
3. ($C_3$-$C_8$)-Cycloalkyl,
4. ($C_6$-$C_{12}$)-Aryl,
5. ($C_7$-$C_{13}$)-Aroyl,
6. ($C_1$-$C_4$)-Alkoxy,
7. ($C_1$-$C_4$)-Alkanoyloxy,
8. ($C_1$-$C_9$)-Heteroaryl,
9. $CO_2R(6)$,
10. Halogen,
11. Cyano,
12. Nitro,
13. NR(17)R(18),
14. Hydroxy,
15. -CO-NH-CHR(19)-$CO_2R(6)$,
16. Sulfo,
17. -$SO_3R(6)$,
18. -$SO_2$-NR(18)-CO-NR(17)R(16), -$SO_2$-NR(18)-CO-O-R(17), -$SO_2N$(CO-O-R(17))-$_2$ oder -$SO_2$-NR(18)-CS-NR(17)R(16),
19. -NR(18)-SO-NR(17)-$SO_2$-$CH_2$-R(18),
20. -$C(CF_3)_2OH$,
21. Phosphonooxy,
22. -$PO_3H_2$,
23. -NH-$PO(OH)_2$,
24. -$S(O)_rR(17)$,
25. -CO-R(20),
26. -CO-NR(17)R(16),
27.

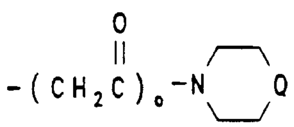

28.

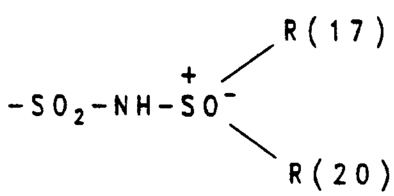

29.

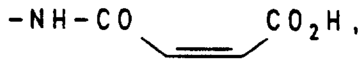

30.

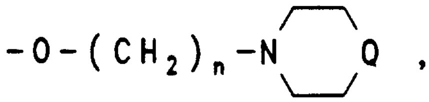

31. 5-Tetrazolyl-NH-CO-,
32. -CO-NH-NH-$SO_2CF_3$,
33.

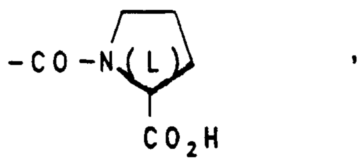

34.

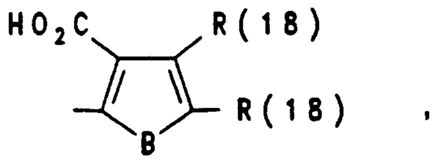

35.

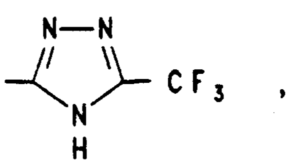

36.

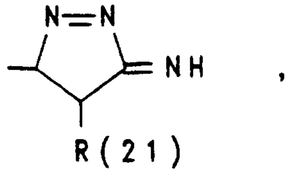

37.

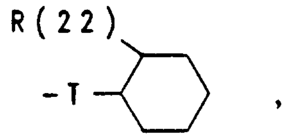

38.

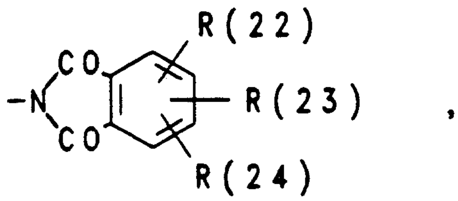

39. -CO-NH-$SO_2$-R(6),
40. -$SO_2$-NH-CO-R(17),
41. den unter 4. definierten Rest, substituiert mit 1 oder 2 gleichen oder verschiedenen Resten aus der Reihe Halogen, Cyano, Nitro, NR(17)R(18) und Hydroxy,
42. R(15) zusammen mit R(14) bedeutet -CO-NH-$SO_2$-;

R(16), R(17) gleich oder verschieden
1. Wasserstoff,
2. ($C_1$-$C_6$)-Alkyl,
3. ($C_3$-$C_8$)-Cycloalkyl,
4. ($C_6$-$C_{12}$)-Aryl, vorzugsweise Phenyl,
5. ($C_6$-$C_{10}$)-Aryl-($C_1$-$C_4$)-Alkyl,
6. ($C_1$-$C_9$)-Heteroaryl, welches teilweise oder vollständig hydriert sein kann, vorzugsweise 2-Pyrimidinyl, 1-Piperidinyl oder Chinuclidinyl,
7. ($C_3$-$C_6$)-Alkenoyl,
8. einen wie unter 4., 5., 6., 9., 14., 15., 16., 18., 19., oder 20. definierten Rest, substituiert mit 1 oder 2 gleichen oder verschiedenen Resten aus der Reihe Hydroxy, Methoxy, Nitro, Cyano, $CO_2R(6)$, Trifluormethyl, -NR(25)R(26) und

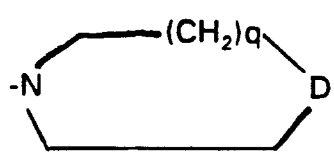

9. ($C_1$-$C_9$)-Heteroaryl-($C_1$-$C_3$)-alkyl, wobei der Heteroarylteil teilweise oder vollständig hydriert sein kann,
10. den unter 2. definierten Rest, woein 1 bis alle H-Atome durch Fluor substituier sind,
11. ($C_2$-$C_6$)-Alkenyl,
12. ($C_3$-$C_8$)-Cycloalkenyl,
13. ($C_3$-$C_8$)-Cycloalkenyl-($C_1$-$C_3$)-alkyl,
14. ($C_3$-$C_8$)-Cycloalkyl-($C_1$-$C_4$)-alkyl,
15. ($C_6$-$C_{10}$)-Aryl-($C_3$-$C_6$)-Alkenyl,
16. ($C_1$-$C_9$)-Hetaryl-($C_3$-$C_6$)-Alkenyl,
17. ($C_3$-$C_6$)-Alkinyl,
18. ($C_6$-$C_{10}$)-Aryl-($C_3$-$C_6$)-Alkinyl,
19. ($C_1$-$C_9$)-Hetaryl-($C_3$-$C_6$)-Alkinyl,
20. einen Rest der Formel

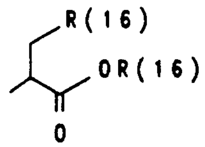 ,

wobei R(16) die Bedeutung von 20. nicht haben kann.
21. R(16)R(17) gemeinsam mit dem sie tragenden N-Atom ein Hetaryl bilden, das auch teilweise oder vollständig hydriert sein kann;

R(18)
1. Wasserstoff,
2. $(C_1-C_6)$-Alkyl,
3. $(C_3-C_8)$-Cycloalkyl,
4. $(C_6-C_{12})$-Aryl-$(C_1-C_6)$-alkyl, vorzugsweise Benzyl,
5. Phenyl oder
6. $(C_1-C_9)$-Heteroaryl;

R(19)
1. Wasserstoff,
2. $(C_1-C_6)$-Alkyl,
3. $(C_3-C_8)$-Cycloalkyl,
4. Phenyl oder
5. Benzyl;

R(20)
1. Wasserstoff,
2. $(C_1-C_6)$-Alkyl,
3. $(C_3-C_8)$-Cycloalkyl,
4. Phenyl-$(CH_2)_q$-,
5. OR(19),
6. NR(25)R(26) oder
7.

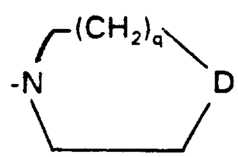

R(21) Cyano, Nitro oder $CO_2R(18)$ bedeutet;
R(22) $CO_2R(6)$ oder $CH_2CO_2R(6)$ bedeutet;
R(23) Wasserstoff, Halogen, $(C_1-C_4)$-Alkyl oder $(C_1-C_4)$-Alkoxy bedeutet;
R(24) Wasserstoff, $(C_1-C_4)$-Alkyl oder Phenyl bedeutet;
R(25) und R(26) gleich oder verschieden sind und
1. Wasserstoff,
2. $(C_1-C_4)$-Alkyl,
3. Phenyl,
4. Benzyl oder
5. $\alpha$-Methylbenzyl

bedeuten;

D NR(23), O oder $CH_2$ bedeutet;
B O, NR(18) oder S bedeutet;
T
1. eine Einfachbindung,
2. -CO-,
3. $-CH_2-$,
4. -O-,
5. -S-,

6. -NR(28)-,
7. -CO-NR(28)-,
8. -NR(28)-CO-,
9. -O-$CH_2$-,
10. -$CH_2$-O-,
11. -S-$CH_2$-,
12. -$CH_2$-S-,
13. -NH-CR(27)R(29)-,
14. -NR(28)-$SO_2$-,
15. -CR(27)R(29)-NH-,
16. -CR(27)R(29)-NH-,
17. -CH = CH-,
18. -CF = CF-,
19. -CH = CF-,
20. -CF = CH-,
21. -$CH_2$-$CH_2$-,
22. -$CF_2$-$CF_2$-,
23. -CH(OR(6))-,
24. -CH(OCOR(19))-,
25.

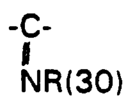

oder

26.

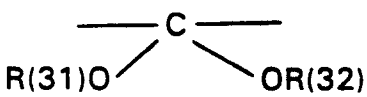

R(27) und R(29) gleich oder verschieden sind und Wasserstoff, ($C_1$-$C_5$)-Alkyl, Phenyl, Allyl oder Benzyl bedeuten;

R(28) Wasserstoff, ($C_1$-$C_6$)-Alkyl, Benzyl oder Allyl bedeutet;

R(30)

1. NR(27)R(28),
2. Ureido,
3. Thioureido,
4. Toluol-4-sulfonyl oder
5. Benzolsulfonylamino

bedeuten;

R(31) und R(32) gleich oder verschieden sind und ($C_1$-$C_4$)-Alkyl bedeuten oder gemeinsam für $(CH_2)_q$- stehen;

Q $CH_2$, NH, O oder S bedeutet;

n eine ganze Zahl von 1 bis 5 ist;

m eine ganze Zahl von 0 bis 3 ist;

o eine ganze Zahl von 1 bis 10 ist;

r Null, 1 oder 2 ist

sowie deren physiologisch verträglichen Salze.

Alkyl, Alkenyl und Alkinyl können geradkettig oder verzweigt sein. Entsprechendes gilt für davon abgeleitete Reste, wie Alkanoyl oder Alkoxy.

Unter Cycloalkyl werden auch alkylsubstituierte Ringe verstanden.

($C_6$-$C_{12}$)-Aryl ist beispielsweise Phenyl, Naphthyl oder Biphenylyl vorzugsweise Phenyl. Entsprechendes gilt für davon abgeleitete Reste, wie Aroyl oder Aralkyl.

Unter ($C_1$-$C_9$)-Heteroaryl werden insbesondere Reste verstanden, die sich von Phenyl oder Naphthyl ableiten, in welchen eine oder mehrere CH-Gruppen durch N ersetzt sind und/oder in welchen mindestens

zwei benachbarte CH-Gruppen (unter Bildung eines fünfgliedrigen aromatischen Rings) durch S, NH oder O ersetzt sind. Weiter kann auch 1 oder beide Atome der Kondensationsstelle bicyclischer Reste (wie im Indolizinyl) ein N-Atom sein.

Dies sind z. B. Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyradiazinyl, Indolyl, Indazolyl, Chinolyl, Isochinolyl, Phthalazinyl, Chinoxalinyl, Chinazolinyl, Chinnolinyl.

Unter dem annellierten Heterobicyclus AH, von dem der Rest A abgeleitet ist, versteht man insbesondere ein bicyclisches Ringsystem mit 8 bis 10 Ringatomen, wovon bis zu 9 Ringatome C-Atome sind, worin zwei nebeneinanderliegende Atome gemeinsame Bestandteile beider Ringe sind. Einer oder beide dieser Ringe leiten sich formel vom Benzol ab, in welchem eine oder mehrere CH-Gruppen durch N, $O^+$, und $S^+$ ersetzt sind und/oder in welchem zwei benachbarte CH-Gruppen (unter Bildung eines fünfgliedrigen aromatischen Rings) durch S, NH oder O ersetzt sind.

A ist beispielsweise ein Rest von Benzothiophen, Benzofuran, Indol, Isoindol, Indazol, Benzimidazol, Chinon, Isochinolin, Phthalazin, Chinoxalin, Chinazolin, Cinnolin, Benzthiazol, Benzthiazol-1, 1-dioxid, Cumarin, Chroman, Benzoxazol, Benzisothiazol, Benzodiazin, Benztriazol, Benztriazin, Benzoxazin, Imidazopyridin, Imidazo-pyrimidin, Imidazo-pyrazin, Imidazo-pyridazin, Imidazo-thiazol, Pyrazolopyridin, Thienopyridin und Pyrrolopyrimidin. Der genannte Heterobicyclus AH kann auch teilweise oder ganz hydriert sein. Vorzugsweise bleibt aber ein Ring von AH aromatisch, wobei ein benzanellierter Heterobicyclus AH besonders bevorzugt ist.

Im Falle von S-haltigen und/oder teilgesättigen Resten kann der Bicyclus z. B. auch oxosubstituiert sein, wie es beim Rest des Benz-1,2,3-triazinon der Fall ist.

Die Verknüpfung von A erfolgt vom isocyclischen oder vom heterocyclischen Teil aus bei q = Null über eine Alkandiyl-Brücke L und bei q = 1 über eine Einfachbindung zur

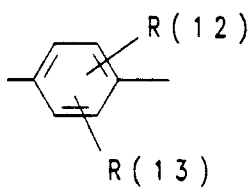

Gruppe

Unter einem Iso- oder Heterocyclus $XH_2$, von denen der mono- oder bicyclische Rest X abgeleitet ist, versteht man beispielsweise einen Rest von Cyclopentan, Cyclohexan, Cycloheptan, Cyclopenten, Cyclohexen, Cyclohepten, Benzol, Naphthalin, Furan, Thiopen, Pyrrol, Pyridin, Pyridazin, Pyrimidin, Piperidin, Piperazin, Morpholin, Indol, Indazol, Oxazol, Isoaxazol, Chinolin, Isochinolin, Benzthiophen, Benzofuran, Benzthiazol, Benzoxazol, Imidazopyridin, Imidazopyrimidin und Rufopyridin.

Halogen steht für Fluor, Chlor, Brom und Jod.

Unter physiologisch verträglichen Salzen von Verbindungen der Formel (II) versteht man sowohl deren organische als auch anorganische Salze, wie sie in Remington's Pharamceutical Sciences, 17. Auflage, Seite 1418 (1985) beschrieben sind. Aufgrund von physikalischer und chemischer Stabilität und Löslichkeit sind für saure Gruppen unter anderem Natrium-, Kalium-, Calcium- und Ammoniumsalze bevorzugt, für basische Gruppen unter anderen Salze mit Salzsäure, Schwelfelsäure, Phosphorsäure, Carbonsäuren oder Sulfonsäuren, wie Essigsäure, Zitronensäure, Benzoesäure, Maleinsäure, Fumarsäure, Weinsäure, p. Toluolsulfonsäure.

Bevorzugt werden Verbindungen der Formel (IV) gegen die genannten Erkrankungen eingesetzt

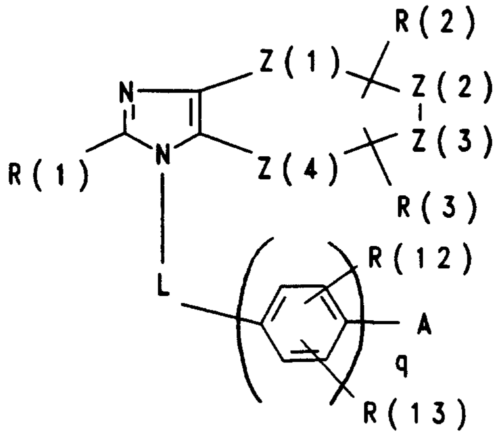

(IV)

wobei die Symbole folgende Bedeutung haben:

Z(1), Z(2), Z(3) und Z(4):

1. $-CH_2-$,
2. -CH=,
3. ein unter 2. definierter Rest, wobei 1 oder 2 Methin-Gruppen durch Stickstoff ersetzt sind; bevorzugt ist Z(4) = N,

R(1)

1. ($C_1C_{10}$)-Alkyl,
2. ($C_3$-$C_{10}$)-Alkenyl,
3. ($C_3$-$C_{10}$)-Alkinyl,
4. ($C_3$-$C_8$)-Cycloalkyl,
5. Benzyl oder
6. Benzyl, das wie oben beschrieben substituiert ist;

R(2) und R(3) gleich oder verschieden stehen für:

1. Wasserstoff,
2. Hydroxy,
3. Halogen,
4. einen linearen oder verzweigten ($C_1$-$C_6$)-Alkylrest, unsubstituiert oder substituiert durch einen oder mehrere gleiche oder verschiedene Substituenten aus der Reihe Halogen, Hydroxy, ($C_1$-$C_4$)-Alkoxy, ($C_1$-$C_4$)-Alkylthio, Mercapto,
5. $-CO_2R(6)$;

T eine Einfachbindung, -O-,-CO-, -NHCO- oder $-OCH_2-$ bedeutet

und die übrigen Reste und Variablen wie oben definiert sind.

Besonders bevorzugt sind Verbindungen der Formel (IV), in welcher

R(1) ($C_1$-$C_7$)-Alkyl, ($C_3$-$C_7$)-Alkenyl oder ($C_3$-$C_7$)-Alkinyl bedeutet;

R(6) Wasserstoff oder ($C_1$-$C_4$)-Alkyl bedeutet;

R(12) und R(13) gleich oder verschieden und Wasserstoff und ($C_1$-$C_4$)-Alkyl bedeuten;

R(14)

1. ($C_1$-$C_4$)-Alkyl,
2. ($C_1$-$C_4$)-Alkoxy,
3. Cyano,
4. Amino,
5. Nitro,
6. Fluor, Chlor oder Brom,
7. ($C_1$-$C_4$)-Heteroaryl-$CH_2$,
8. ($C_1$-$C_4$)-Alkanoyloxy,
9. ($C_1$-$C_4$)-Alkanoyl,
10. Benzoyl oder
11. Tetrazolyl bedeutet;

R(15)

1. ($C_1$-$C_4$)-Alkyl,

2. $(C_6-C_{12})$-Aryl,
3. $(C_1-C_3)$-Alkanoyloxy,
4. $(C_1-C_4)$-Alkoxy,
5. $(C_1-C_9)$-Heteroaryl, vorzugsweise 5-Tetrazolyl,
6. Cyano,
7. Nitro,
8. Hydroxy,
9. $SO_3R(6)$,
10. Chlor, Brom,
11. $CO_2R(6)$,
12. CO-NH-R(19),
13. CO-R(20),
14. $SO_2$-NR(18)-CO-NR(17)R(16),
15. $SO_2$-NR(18)-CO-O-R(17) oder $SO_2N(CO-OR(17))_2$,
16. CO-CHR(19)-$CO_2H$,
17. $(C_1-C_4)$-Alkyl-$CO_2H$,
18. NH-CO-NH-$SO_2$-$CH_2$-R(19),
20.

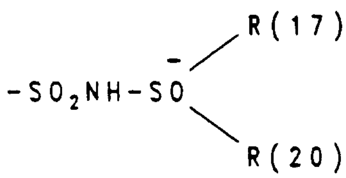

,

21.

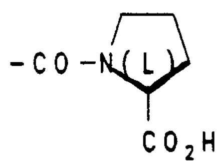

,

22.

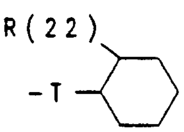

oder
23. R(14) mit R(15) zusammen -CO-NH-$SO_2$;

L -$CH_2$-;
R(18) Wasserstoff;
R(25) und R(26) unabhängig voneinander Wasserstoff oder $(C_1-C_4)$-Alkyl

bedeuten,
sowie deren physiologisch verträgliche Salze.

Das Verfahren zur Herstellung von Verbindungen der Formel (II) ist dadurch gekennzeichnet, daß man Verbindungen der Formel (IIIb)

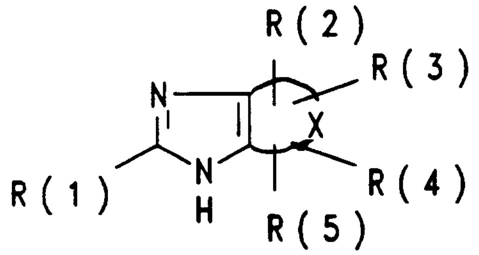

(IIIb)

worin R(1), R(2), R(3), R(4), R(5) und X wie oben definiert sind, alkyliert mit Verbindungen der Formel (IVb)

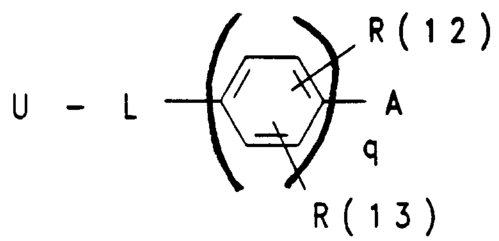

(IVb)

worin L, q, R(12), R(13) und A wie oben definiert sind und U für eine Fluchtgruppe steht, gegebenenfalls temporär eingeführte Schutzgruppen wieder abgespaltet und die erhaltenen Verbindungen der Formel (II) gegebenenfalls in ihre physiologisch verträglichen Salze überführt werden.

Geeignete Fluchtgruppen U sind vorzugsweise nucleofuge Gruppen (vgl. Angew. Chem. 72 (1960)) wie Halogen, o-Toluolsufonat, Mesylat oder Triflat.

Verfahren zur Herstellung der Vorstufen der Formel (IIIb) sind u. a. bekannt aus US 4 880 804, DE 3 911 603, EP-A-399 731, EP-A-399 732, EP-A-400 835, EP-A-400 974, EP-A-415 886, EP-A-420 237, EP-A-425 921 und EP-A-434 038.

Zur Alkylierung der Verbindungen der Formel (IIIb) sind z. B. entsprechende Benzylhalogenide, -tosylate, -mesylate oder -triflate oder entsprechende Alkylhalogenide, -tosylate, -mesylate oder -triflate geeignet.

Die Darstellung dieser Verbindungen erfolgt in an sich bekannter Weise, beispielsweise durch Halogenierung der entsprechenden Methylvorstufen. Hierzu wird vorzugsweise N-Bromsuccinimid eingesetzt, siehe z. B. J. Org. Chem. 44, 4733 (1979) und Helv. Chim. Acta 62, 2661 (1979).

Die Synthese der Benzimidazol-, Benzthiophen-, Imidazo-pyridin- und Imidazo-pyrimidin-Derivate mit $CH_3$-Gruppe am Kern erfolgte unter anderem nach R. P. Dickson et al. in J. Med. Chem. 29, 1937 (1986), E. Abignente et al. in J. Heterocyclic Chem. 26, 1875 (1989), A. Koubsack et al. in J. Org. Chem. 41, 3399 (1976) und nach F. Santer et al. in Mh. Chem. 99, 715 (1968).

Die Biphenylderivate können z. B. ausgehend von Arylboronsäurederivaten durch Kopplung mit substituierten Arylhalogeniden mit Übergangsmetallkatalysatoren, insbesondere Palladium synthetisiert werden. Entsprechende Reaktionen werden von R. B. Miller et al. (Organometallics 1984, 3, 1261) oder von A. Zuzuki et al. (Synthetic Commun. 11 (7), 513 (1981)) beschrieben.

Die Sulfonurethan-Derivate der Formel (II) können aus entsprechenden Sulfonamiden der Formel (III) durch Umsetzung mit Chlorkohlensäureestern oder durch Umsatz mit Dimethyldicarbonat und Basen wie z. B. Kaliumvarbonat in inerten Lösungsmitteln bei Temperaturen bis zum Siedepunkt des entsprechenden Lösungsmittels erhalten werden.

Die Sulfonylharnstoff-Derivate der Formel (II) sind wahlweise entweder aus den entsprechenden Sulfonamiden der Formel (II) durch Umsetzung mit Isocyanaten oder mit 2,2,2-Trichloracetamid-Derivaten eines geeigneten Amins in inerten, hochsiedenden Lösungsmitteln wie z. B. DMSO oder aus Sulfonylurethanen der Formel (II) durch Einwirkung des entsprechenden Amins in einem inerten, hochsiedenden Lösungsmittel wie z. B. Toluol bei Temperaturen bis zum Siedepunkt des jeweiligen Lösungsmittels darstellbar.

Der Sulfonamid-Rest kann, falls erforderlich, ausgehend von einer Aminogruppe mittels Meerwein-Umlagerung hergestellt werden. Hierfür wird das Hydrochlorid des Amins zuerst diazotiert und dann in Gegenwart eines Kupferkatalysators mit Schwefeldioxid in Eisessig umgesetzt. Nachfolgende Einwirkung von Ammoniak führt zur Sulfonamidogruppe.

Die Alkylierung erfolgt nach prinzipiell bekannter Verfahren in analoger Weise.

Die Imidazo-anellierten Derivate der Formel (IIIb) werden z. B. in Gegenwart einer Base metalliert. Bevorzugte Basen sind Metallhydride der Formel MH wie Lithium-, Natrium- oder Kaliumhydrid in beispielsweise DMF oder DMSO als Lösungsmittel oder Metallalkoxide der Formel MOR, wobei R für Methyl, Ethyl, t-Butyl steht und die Reaktion in dem entsprechenden Alkohol, DMF oder DMSO durchgeführt wird. Die so gebildeten Salze der Imidazo-Derivate dwerden in einem aprotischen Lösungsmittel wie DMF oder DMSO gelöst und mit einer geeigneten Menge Alkylierungsreagenz versetzt.
Eine alternative Möglichkeit zur Deprotonierung der Imidazol-Derivate stellt z. B. die Umsetzung mit Kaliumcarbonat in DMF oder DMSO dar.
Die Umsetzungen werden bei Temperaturen unterhalb Zimmertemperatur bis zum Siedepunkt des Reaktiongemisches, vorzugsweise zwischen + 20 °C und dem Siedepunkt des Reaktionsgemisches während etwa 1 bis 10 Stunden durchgeführt.
Bei den Verbindungen der allgemeinen Formeln (I), (II), (III) und (IV) haben sich diejenigen Verbindungen als bezüglich ihres Metabolismus, insbesondere im Menschen, besonders günstig erwiesen, die als Substituenten des Biphenylsystems eine Sulfoharnstoff-Gruppierung wie z. B. $SO_2$-$NR^7$-CO-$NR^6R^9$ enthalten.

Insbesondere von Interesse hinsichtlich ihrer therapeutischen Wirkung bei der Behandlung von Koronaren Herzerkrankungen und den übrigen genannten Krankheiten sind auch die folgenden Verbindungen:

1. 2-n-Butyl-1-[(2'n-propylaminocarbonylaminosulfonyl-biphenyl-4-yl)methyl]-4-methylthio-imidazol-5-carbonsäureethylester
2. 2-n-Butyl-1-[(2'-n-propylaminocarbonylaminosulfonyl-biphenyl-4-yl)methyl]-4-methylthio-imidazol-5-carbonsäure
3. 2-n-Propyl-1-[(2'-n-propylaminocarbonylaminosulfonyl-biphenyl-4-yl)methyl]-4-methylthio-imidazol-5-carbonsäureethylester
4. 2-n-Propyl-1-[(2'n-propylaminocarbonylaminosulfonyl-biphenyl-4-yl)methyl]-4-methylthio-imidazol-5-carbonsäure
5. 3-[(2'Allylaminocarbonylaminosulfonyl-biphenyl-4-yl)methyl]-2-ethyl-7-methyl-imidazo[4,5-b]pyridin
6. 5,7-Dimethyl-3-[[2'-methoxycarbonylaminosulfonyl-biphenyl-4-yl)methyl]-2-ethyl-imidazo[4,5-b]pyridin
7. 2-n-Butyl-4-chloro-5-hydroxymethyl-1-[(2'-(1H-tetrazol-5-yl)-biphenyl-4-yl)-methyl]-imdidazol-kaliumsalz, bekannt aus EP-A 324 377
8. 5,7-Dimethyl-2-ethyl-3-[(2'-tetrazoyl-biphenyl-4-yl)methyl]-imidazol-[4,5-b]pyridin bekannt aus EP-A 399 731, EP-A 400 974 u. N. B. Mantlo, J. Med. Chem. 34, 1991, 2919-2922
9. 2-n-Butyl-4-chloro-1-[(2'(1H-tetrazol-5yl)-biphenyl-4-yl)-methyl]-imidazol-5-carbonsäure (siehe P.C. Wong et al.l, J. Pharmacol. Exp. Ther. 252, 711-718, 1990)

sowie deren physiologisch verträglichen Salze wie die entsprechenden Mono- bzw. Di-Kaliumsalze.

Darüber hinaus ist die Behandlung von koronaren Herzerkrankungen und den übrigen genannten Erkrankungen auch möglich durch die Verwendung von Angiotensin-II-Rezeptorantagonisten der allgemeinen Formeln VI - XX, die im jeweils angegebenen Stand der Technik beschrieben werden.

(VI) 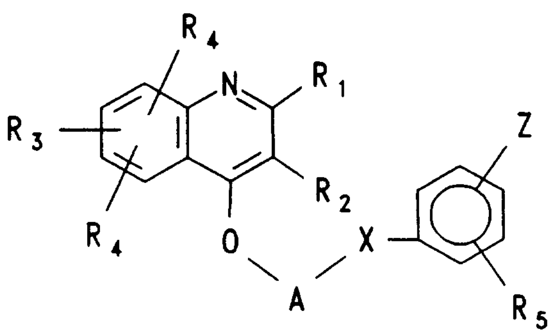 ,

beschrieben in EP-A 412 848,

(VII) 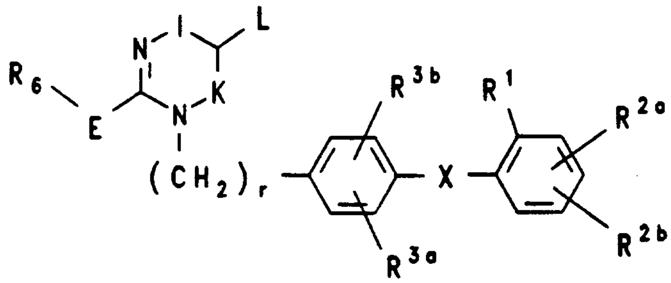 ,

beschrieben in EP-A 411 766,

(VIII) 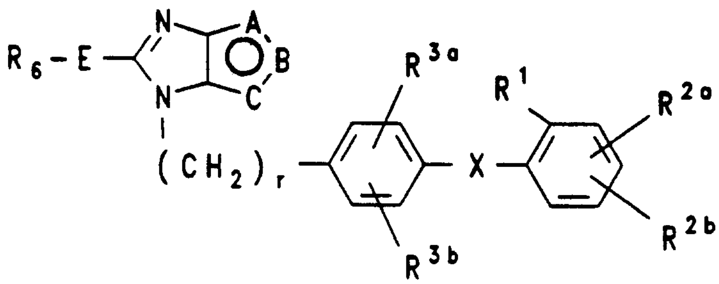 ,

beschrieben in EP-A 407 102,

(IX) 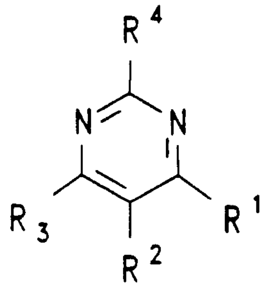 ,

beschrieben in EP-A 424 317,

(X) 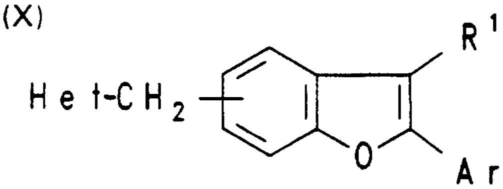 ,

beschrieben in EP-A 434 249

(XI)

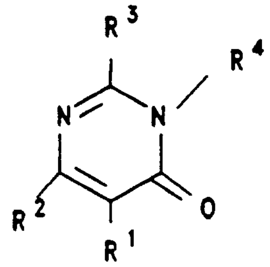

,

beschrieben in EP-A 435 827

(XII)

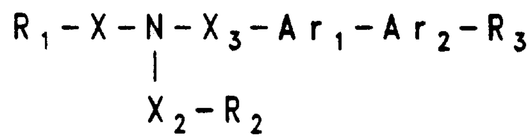

,

beschrieben in EP-A 443 983,

(XIII)

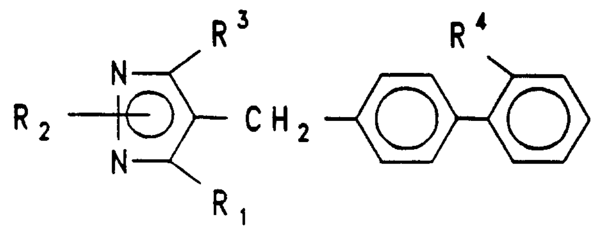

,

beschrieben in EP-A 446 062,

(XIV)

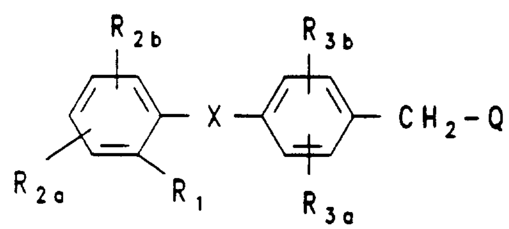

,

beschrieben in WO 91-15209

(XV)

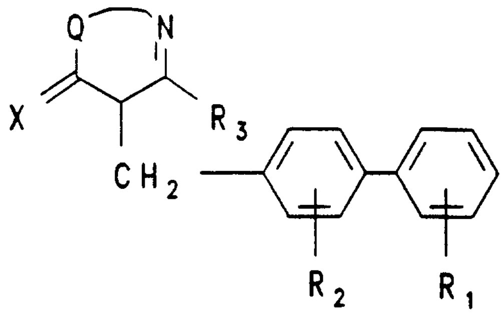

,

beschrieben in WO 91-14679,

(XVI)

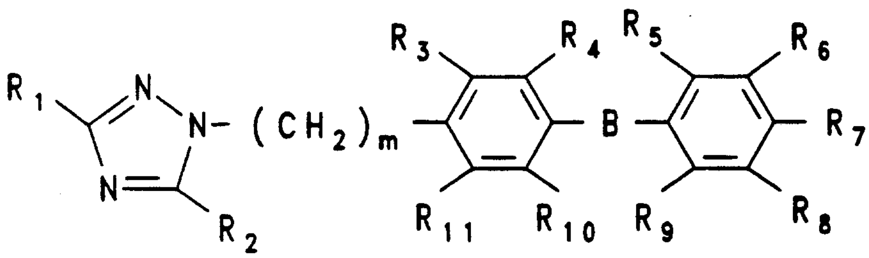

,

beschrieben in WO 91-17148

(XVII)

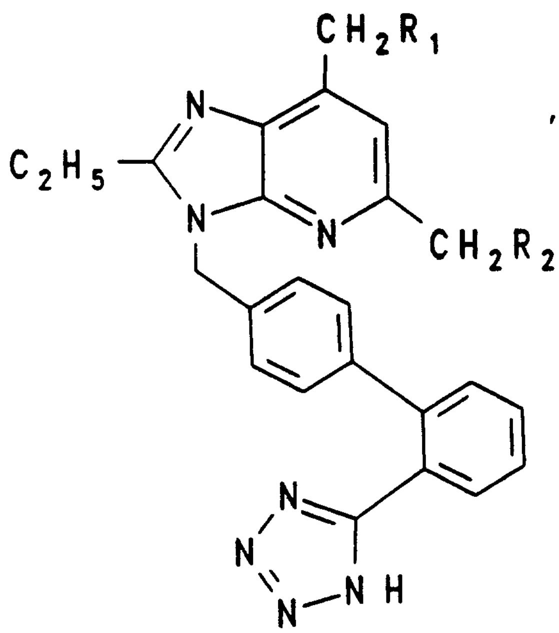

,

beschrieben in EP-A 456 510

(XVIII)

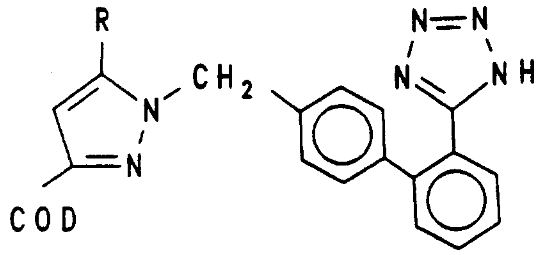

,

beschrieben in EP-A 411 507

(XIX)

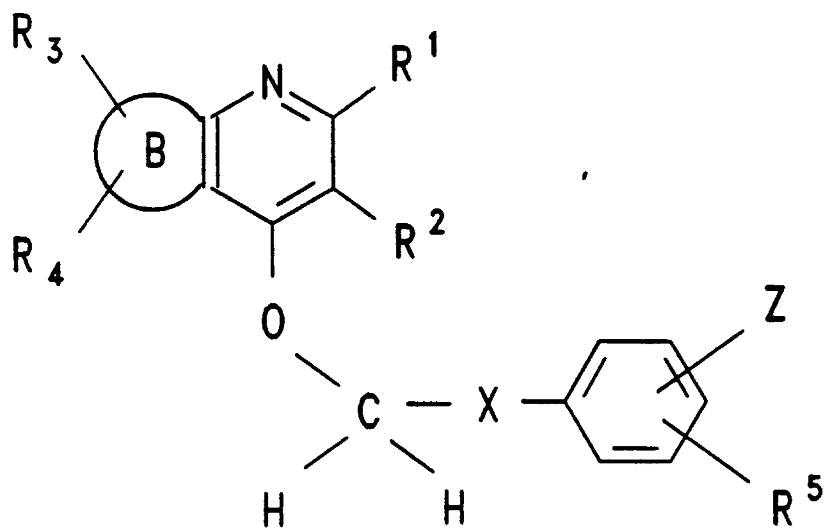

,

beschrieben in WO 91-07404 und

(XX)

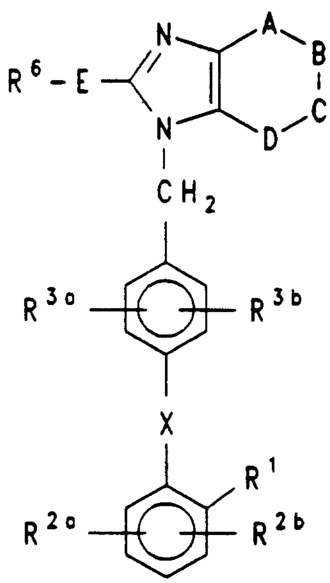

,

beschrieben in EP-A 400 974

In Ausübung der erfindungsgemäßen Methode können die oben beschriebenen Antagonisten des Angiotensin-II-Rezeptors an Säugern wie Affen, Hunden, Katzen oder Ratten, insbesondere aber beim Menschen angewendet werden.

Die für die erfindungsgemäßen Verwendung geeigneten Verbindungen werden zweckmäßig in üblicher Weise in pharmazeutische Präparate eingearbeitet. Sie können in die üblichen Verabreichungsformen wie Kapseln, Tabletten, Dragees, Lösungen, Salben, Emulsionen und auch in Depot-Form gebracht werden. Der Wirkstoff kann gegebenenfalls auch in mikroverkapselter Form vorliegen. Die Präparate können physiologisch verträgliche organische bzw. anorganische Hilfs- bzw. Zusatzstoffe, beispielsweise Granulierstoffe, Kleb- und Bindemittel, Gleitmittel, Suspendiermittel, Lösungsmittel, antibakterielle Mittel, Netzmittel und Konservierungsmittel enthalten.

Die erfindungsgemäße Behandlung kann sowohl über die Schleimhäute als auch parenteral erfolgen. Orale und parenterale (wie i.v. oder i.m.) Anwendungsformen werden bevorzugt.

Für eine orale Anwendungsform werden die aktiven Verbindungen mit den dafür üblichen Zusatzstoffen wie Trägerstoffen, Stabilisatoren oder inerten Verdünnungsmittel vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumcarbonat, Kaliumsphosphat, Milchzucker, Glucose oder Stärke, insbesondere Maistärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträglichen Salze gewünschtenfalls mit den dafür üblichen Substanzen wie Lösungsvermittler, Emulgator oder weitere Hilfsstoffe in Lösung, Suspensionen oder Emulsionen gebracht. Als Lösungsmittel für die aktven Kombinationen und die entsprechenden physiologisch verträglichen Salze kommen z.B. in Frage: Wasser, physiologische Kochsalzlösungen oder Alkohole, z. B. Ethanol, Propandiol oder Glycerin, daneben auch Zuckerlösungen wie Glucose- oder Mannitlösungen oder auch eine Mischung aus den verschiedenen genannten Lösungsmitteln.

Die beschriebenen Verbindungen werden bevorzugt in Dosen von 0,1 bis 100 mg/kg verabreicht, wobei im einzelnen vorzugsweise 0,1 bis 50 mg, insbesondere 1 bis 30 mg ein- bis dreimal täglich gegeben werden.

Die Wirksamkeit der beschriebenen Verbindungen zur Behandlung und Prophylaxe von koronaren Herzerkrankungen, des septischen Schocks, von kognitiven und erektiven Dysfunktionen sowie Nieren- und Immunerkrankungen kann aus dem Versuch zur in vitro-Bildung von cGMP an Endothelzellen der Rinderaorta abgeleitet werden.

Beispiel 1

Es wurde folgende Untersuchung mit 2-n-Butyl-4-chloro-1-[(2'-(1H-tetrazol-5-yl)-biphenyl-4-yl)methyl]-imidazol-5-carbonsäure (T) durchgeführt:

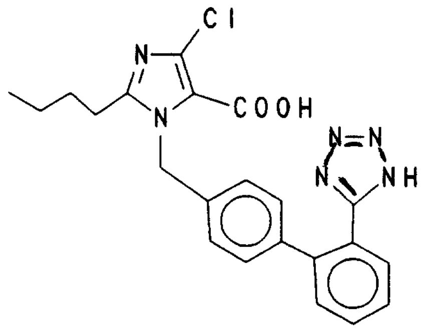

(T)

Versuch

Wirkung von Angiotensin-II an Endothelzellen der Rinderaorta unter Vorinkubation mit dem $AT_1$-Rezeptorantagonisten 2-n-Butyl-4-chloro-1-[(2'-(1H-triazol5-yl)-biphenyl-4-yl)methyl]-imidazol-5-carbonsäure

Methode

Primäre kultivierte Endothelzellen der Rinderaorta werden mit $10^{-7}$-$10^{-4}$ mol/l Angiotensin II (ANG II) 3 Minuten lang inkubiert und die Konzentrationsänderungen von zyklischem GMP (cGMP) mittels eines Radioimmunassays in pmol/mg Protein bestimmt (siehe Kurve A in Fig. 1).

Die Messung wird nach Vorinkubation (5 Minuten) der Endothelzellen mit

a) einem stereospezifischen Hemmstoff der NO-Synthese (N*-Nitro-L-Arginin L-NNA ($10^{-5}$ mol/l)) (siehe Kurve B in Fig. 1)

b) einem spezifischen Bradykinin-Antagonisten,

dem Decapeptid (HOE 140) H-D-Arg-Arg-Pro-Hyp-Gly-Thi-Ser-D-Tic-Oic-Arg-OH, (siehe Hock et al., Br. J. Pharmacol. 102 (1991) 769-773 und Wirth et al., Br. J. Pharmacol. 102 (1991) 774-777), ($10^{-7}$ mol/l)) (siehe Kurve C in Fig. 1),

c) dem $AT_2$-Rezeptor spezifischen Angiotensin-II-Rezeptorantagonisten

PD 123 177 ($10^{-5}$ mol/l) (bekannt aus J. Med. Chem. 1991, 34, 3248-3260) (siehe Kurve E in Fig. 1)

d) und dem $AT_1$-spezifischen Angiotensin-II-Rezeptor-Antagonisten der Formel (T) in der Konzentration von $10^5$ mol/l (siehe Kurve D in Fig. 1) wiederholt. Dabei ergaben sich Versuchsergebnisse, die in Figur 1 dargestellt sind.

Aus diesen Messungen folgt, daß die durch Angiotensin-II stimulierte Bildung von zyklischen GMP (cGMP) an Endothelzellen über den $AT_2$-Rezeptor-Subtyp der Angiotensin II-Rezeptoren vermittelt ist.

Eine erhöhte Produktion von zyklischem GMP geht jedoch einher mit einer erhöhten endothelialen Nitriloxid(NO)-Bildung (siehe z. B. Moncada et al., Pharmacological reviews 43, No. 2 (1991) 109-142). Eine selektive Hemmung des $AT_1$-Subtyps von Angiotensin II-Rezeptoren mit den beschriebenen Verbindungen erhöht sowohl durch die $AT_1$-Rezeptor-Blockade selbst als auch durch die mit ihr korrelierte kompensatorische Renin-Ausschüttung aus der Niere die an den $AT_2$-Rezeptor-Subtyp bindende Angiotensin-II-Menge. Nach B. Bankenburg et al. (Hypertension 18 (1991) 278-289) erhöht sich nach der Behandlung von SHR (spontan hypertensiven Ratten) z. B. mit der Verbindung 2-n-Butyl-4-chloro-5-hydroxymethyl-1-[(2'-(1H-tetrazol-5-yl)-biphenyl-4-yl)methyl]-imidazol-Kaliumsalz in einer Dosis von 10 mg/kg/Tag für 7 Tage die Plasmokonzentration von Renin und Angiotensin II um den Faktor 7 bzw. 10 (negatives feedback).

Die so erhöhten Angiotensin II-Spiegel stimulieren die endotheliale cGMP- bzw. NO-Produktion, womit prinzipiell alle durch Erhöhung des NO-Spiegels positiv beeinflußbare Krankheiten behandelbar werden. Dieses sind u.a. die eingangs beschriebenen koronaren Herzerkrankungen.

Die folgenden Beispiele geben die Anwendungsformen zur Behandlung von den genannten Erkrankungen nach der erfindungsgemäßen Methode an. Die Verbindungen der allgemeinen Formeln I - IV, aber auch die Verbindungen VI bis XX, können analog den Beispielen in die entsprechenden Anwendungsformen

gebracht und zur Therapie von durch NO beeinflußbare Erkrankungen verwandt werden.

Beispiel 2

Es wurde folgender Versuch mit 2-n-Propyl-1[(2'-n-propylaminocarbonyl-aminosulfonyl-biphenyl-4-yl)-methyl]-4-methylthio-imidazol-5-carbonsäure (U) durchgeführt:

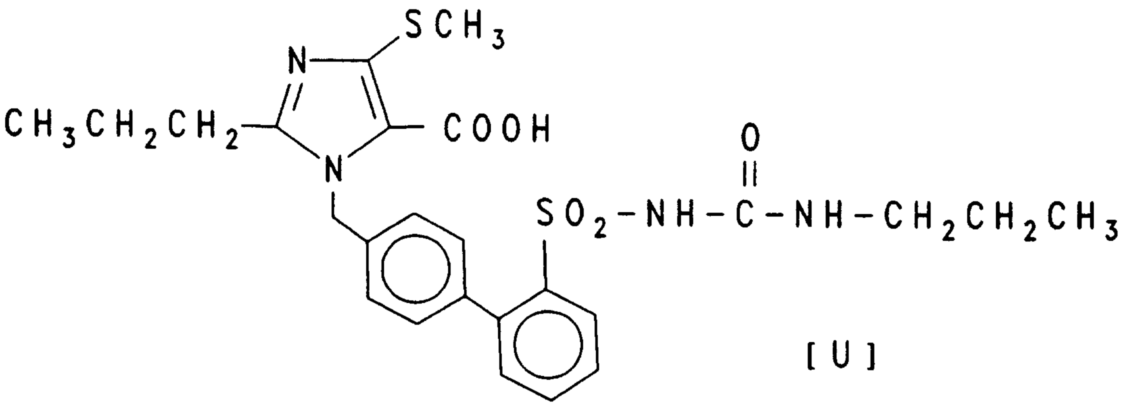

[U]

Versuch

Wirkung des $AT_1$-Rezeptorantagonisten (U) an Endotholzellen der Rinderaorta unter Vorinkubation mit Angiotensin II.

Methode

Primäre kultivierte Endotholzellen der Rinderaorta werden mit $10^{-4}$ mol/l Angiotensin II 3 Minuten lang inkubiert. Die Konzentrationsänderungen von zyklischem GMP (cGMP, zyklisches Guanosinmonophosphar) mittels eines Radioinnunassays bestimmt. Der stimulatorische Effekt von Angiotensin II wird als 100 %-Wert genommen.
Die Messung wird wie in Figur 2 gezeigt nach Vorinkubation (5 Minuten) der Endotholzelle mit ansteigenden Konzentrationen von $10^{-8}$ bis $10^{-4}$ mol/l des $AT_2$-spezifischen Rezeptorantagonisten PD 123 177 (Kurve A) und mit ansteigenden Konzentrationen von $10^{-8}$ bis $10^{-4}$ mol/l des $AT_1$ -selektiven Angiotensin-II-Rezeptorantagonisten der Formel (U) (Kurve B in Fig. 2) wiederholt.

Das resultierende Versuchsergebnis ist in Figur 2 dargestellt.

Beispiel 3

Es wurde folgender Versuch mit dem Imidazopyridin (V) durchgeführt:
3-[(2'-Allylaminocarbonylaminosulfonyl-biphenyl-4-yl)methyl]-2-ethyl-7-methyl-imidazo[4.5-b]pyridin

(V)

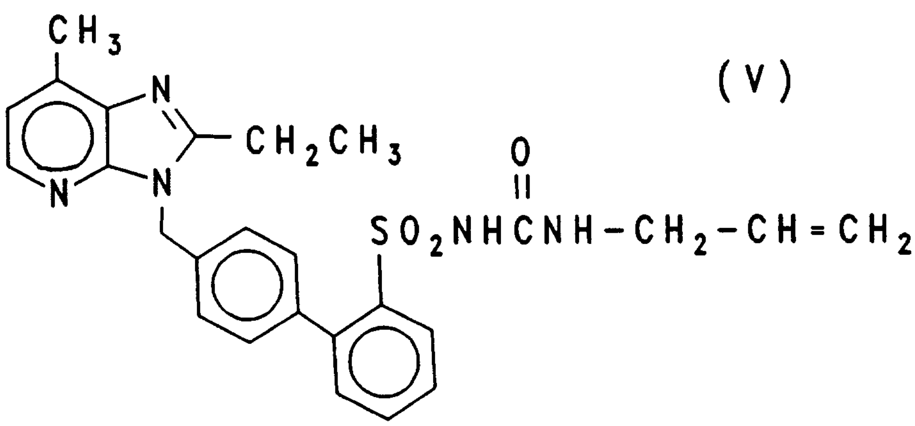

Versuch

Wirkung des $AT_1$-Rezeptorantagonisten (V) an Endotholzellen der Rinderaorta unter Vorinkubation mit Angiotensin II.

Methode

Primäre kultivierte Endotholzellen der Rinderaorten werden mit $10^{-4}$ mol/l Angiotensin II 3 Minuten lang inkubiert. Die Konzentrationsänderungen von zyklischem GMP (cGMP, zyklisches Guanosinmonophosphat) mittels eines Radioimmunassays bestimmt. Der stimulatorische Effekt von Angiotensin II wird als 100 %-Wert genommen.
Die Messung wird nach Vorinkubation (5 Minuten) der Endothelzelle mit ansteigenden Konzentrationen von $10^{-8}$ bis $10^{-4}$ mol/l des $AT_2$-spezifischen Rezeptorantagonisten PD 123 177 (siehe Kurve A) und mit ansteigenden Konzentrationen von $10^{-8}$ bis $10^{-4}$ mol/l des $AT_1$-selektiven Angiotensin II-Rezeptorantagonisten der Formel (V) (siehe Kurve C) wiederholt.
Das resultierende Versuchsergebnis ist in Figur 2 dargestellt.

Beispiel 4

Herstellung eines Mittels zur oralen Anwendung:
1000 Tabletten, die je 20 mg 2-n-Propyl-1-[(2'n-propylamino-carbonylamino-sulfonyl-biphenyl-4-yl)methyl]-4-methylthio-imidazol-5-carbonsäure enthalten, werden mit den folgenden Hilfsmittel hergestellt:

| | |
|---|---|
| 2-n-Propyl-1-[(2'n-propylaminocarbonylaminosulfonyl-biphenyl-4-yl)methyl]-4-methylthio-imidazol-5-carbonsäure | 20,0 g |
| Maisstärke | 140,0g |
| Gelantine | 7,5 g |
| Mikrokristalline Cellulose | 2,5 g |
| Magnesiumstearat | 2,5 g |

2-n-Propyl-1-[(2'-propylaminocarbonylaminosulfonyl-biphenyl-4-yl)-methyl]-4-methylthio-imidazol-5-carbonsäure und Maisstärke werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das enstandene Granulat wird zu 1000 Tabletten gepreßt, wobei jede Tablette 20 mg des Angiotensin II-Rezeptorantagonisten enthält.

Analog werden Tabletten hergestellt, die statt des oben genannten Wirkstoffs (U) den in Beispiel 1 genannten Wirkstoff (T) bzw. den in Beispiel 3 genannten Wirkstoffs (V) enthalten.

Beispiel 5

Analog Beispiel 4 werden 1000 Tabletten hergestellt, die je 3 mg 2-n-Propyl-1-[(2'-n-propylaminocarbonylaminosulfonyl-biphenyl-4-yl)methyl]-4-methylthio-imidazol-5-carbonsäure enthalten, in dem man von dieser Verbindung 3 g in den in Beispiel 4 beschriebenen Ansatz verwendet.

Beispiel 6

Gelatine-Kapseln, die je 20 mg 2-n-Propyl-1-[(2'-n-propylaminocarbonylaminosulfonyl-biphenyl-4-yl)-methyl]-4-methylthio-imidazol-5-carbonsäure enthalten, werden mit der folgenden Mischung gefüllt:

| | |
|---|---|
| 2-n-Propyl-1-[(2'-n-propylaminocarbonylaminosulfonyl-biphenyl-4-yl)-methyl]-4-methylthio-imidazol-5-carbonsäure | 20 mg |
| Kaliumstearat | 1 mg |
| Lactose | 214 mg |

Analog werden Gelantive-Kapseln mit den Wirkstoffen (T) und (V) hergestellt.

Beispiel 7

Analog Beispiel 3 werden unter Verwendung von 3 mg Wirkstoff Gelatine-Kapseln hergestellt, die je 3 mg 2-n-Propyl-1-[(2'-n-propylaminocarbonylaminosulfonyl-biphenyl-4-yl)methyl]-4-methylthio-imidazol-5-carbonsäure enthalten.

Beispiel 8

Die Herstellung einer Injektionslösung zur Behandlung von u.a. koronaren Herzerkrankungen wird im folgenden beschrieben:

| | |
|---|---|
| 2-n-Propyl-1-[(2'n-propylaminocarbonylaminosulfonyl-biphenyl-4-yl-)methyl]-4-methylthio-imidazol,5-carbonsäure | 1 g |
| Methylparaben | 5 g |
| Propylparaben | 1 g |
| Natriumchlorid | 25 g |
| Wasser für Injektionen | 5 l |

2-n-Propyl-1-[(2'-n-propylaminocarbonylaminosulfonyl-biphenyl-4-yl)-methyl]-4-methylthio-imidazol-5-carbonsäure, die Konservierungsstoffe und Natriumchlorid werden in 3 l Wasser für Injektionen gelöst und auf 5 l mit Wasser für Injektionen aufgeführt. Die Lösung wird steril gefiltert und aseptisch in vorsterilisierte Flaschen gefüllt, die mit sterilisierten Gummikappen verschlossen werden. Jede Flasche enthält 5 ml Lösung.

Analog werden Injektionslösungen hergestellt, die die Wirkstoffe (T) bzw. (V) enthalten.

Beispiel 9

Tabletten werden wie in Beispiel 4 beschrieben hergestellt, mit der Ausnahme, daß anstelle von 2-n-Propyl-1-[(2'-n-propylaminocarbonylsulfonyl-biphenyl-4-yl)methyl]-4-methylthio-imidazol-5-carbonsäure eine der folgenden Verbindungen eingesetzt wird:
2-n-Propyl-1-[(2'-n-propylaminocarbonylaminosulfonyl-biphenyl-4-yl)methyl]-4-methylthio-imidazol-5-carbonsäureethylester oder
2-n-Butyl-1-[(2'-n-propylaminocarbonylaminosulfonyl-biphenyl-4-yl)methyl]-4-methylthio-imidazol-5-carbonsäureethylester,
2-n-Butyl-1-[(2'-n-propylaminocarbonylaminosulfonyl-biphenyl-4-yl)methyl]-4-methylthio-imidazol-5-carbonsäure,
5,7-Dimethyl-3-[(2'-methoxycarbonylaminosulfonyl-biphenyl-4-yl)methyl]-2-ethyl-imidazo[4,5-b]pyridin,
2-n-Butyl-4-chloro-5-hydroxymethyl-1-[(2'-(1H-tetrazol-5-yl)-biphenyl-4-yl)methyl]-imidazol
5, 7-Dimethyl-2-ethyl-3-[(2'-tetrazol-biphenyl-4-yl)methyl]-imidazo[4,5-b]pyridin,
oder die entsprechenden Mono- bzw. Di-Kaliumsalze angewendet werden.

Beispiel 10

Eine Injektionslösung wird analog der in Beispiel 5 beschriebenen Vorschrift hergestellt mit der Ausnahme, daß anstelle von Tabletten werden wie in Beispiel 1 beschrieben hergestellt, wobei jedoch anstelle von 2-n-Propyl-1-[(2'-n-propylaminocarbonylsulfonyl-biphenyl-4-yl)methyl]-4-methylthio-imidazol-5-carbonsäure folgende Substanzen eingesetzt werden:
2-n-Propyl-1-[(2'-n-propylaminocarbonylaminosulfonyl-biphenyl-4-yl)methyl]-4-methylthio-imidazol-5-carbonsäureethylester,
2-n-Butyl-1-[(2'-n-propylaminocarbonylaminosulfonyl-biphenyl-4-yl)methyl]-4-methylthio-imidazol-5-carbonsäureethylester,
2-n-Butyl-1-[(2'-n-propylaminocarbonylaminosulfonyl-biphenyl-4-yl)methyl]-4-methylthio-imidazol-5-carbonsäure,
5,7-Dimethyl-3-[(2'-methoxycarbonylaminosulfonyl-biphenyl-4-yl)methyl]-2-ethyl-imidazo[4,5-b]pyridin,
2-n-Butyl-4-chloro-5-hydroxymethyl-1-[(2'-(1H-tetrazol-5-yl)-biphenyl-4-yl)methyl]-imidazol
5,7-Dimethyl-2-ethyl-3-[(2'-tetrazol-biphenyl-4-yl)methyl]-imidazo[4,5-b]pyridin
oder die entsprechenden Mono- bzw. Di-Kaliumsalze angewendet werden.

## Patentansprüche

1. Verwendung von Antagonisten für Angiotensin-II-Rezeptoren des $AT_1$-Subtyps zur Herstellung eines Arzneimittels zur Behandlung und Prophylaxe von koronaren Herzerkrankungen.

2. Verwendung von Angiotensin-II-Antagonisten gemäß Anspruch 1, dadurch gekennzeichnet, daß heterocyclische Verbindungen der Formel (I) eingesetzt werden

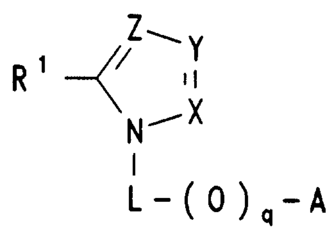

(I)

in welcher die Symbole folgenden Bedeutung haben:

a) X, Y und Z gleich oder verschieden sind und N oder $CR^2$

b) $R^1$

1. $(C_1\text{-}C_{10})$-Alkyl,
2. $(C_3\text{-}C_{10})$-Alkenyl,
3. $(C_3\text{-}C_{10})$-Alkinyl,
4. $-OR^3$
5. $(C_3\text{-}C_8)$-Cycloalkyl,
6. $(C_4\text{-}C_{10})$-Cycloalkylalkyl,
7. $(C_5\text{-}C_{10})$-Cycloalkylalkenyl,
8. $(C_5\text{-}C_{10})$-Cycloalkylalkinyl,
9. $-(CH_2)_m\text{-}B\text{-}(CH_2)_nR^4$,
10. -Benzyl
11. einem wie unter b) 1., 2., 3. oder 9. definierten Rest, der mit $CO_2R^3$ monosubstituiert ist,
12. einem wie unter b) 1., 2., 3. oder 9. definierten Rest, worin 1 bis alle H-Atome durch Fluor substituiert sind, oder
13. den unter b) 10. definierten Rest, der am Phenyl mit 1 oder 2 gleichen oder verschiedenen Resten aus der Reihe Halogen, $(c_1\text{-}c_4)$-Alkoxy und Nitro substituiert ist,

c) $R^2$

1. Wasserstoff,
2. Halogen,
3. Nitro,
4. $C_vF_{2v+1}$
5. Pentafluorphenyl
6. Cyano,
7. $-O\text{-}R^6$
8. Phenyl
9. Phenyl-$(C_1\text{-}C_3)$-alkyl
10. $(C_1\text{-}C_{10})$-Alkyl
11. $(C_3\text{-}C_{10})$-Alkenyl
12. Phenyl-$(C_2\text{-}C_6)$-Alkenyl
13. 1-Imidazolyl-$(CH_2)_m$-,
14. 1,2,3-Triazolyl-$(CH_2)_n$-,
15. Tetrazolyl-$(CH_2)_m$-,
16. $-(CH_2)_{o-1}\text{-}CHR^7\text{-}OR^5$,
17. $-(CH_2)_o\text{-}O\text{-}CO\text{-}R^3$,
18. $-(CH_2)_o\text{-}S\text{-}R^6$,
19. $-S(O)_r\text{-}R^{19}$,
20. $-CH=CH\text{-}(CH_2)_mCHR^3\text{-}OR^6$,
21. $-CH=CH\text{-}(CH_2)_m\text{-}CO\text{-}R^8$,

22. $-CO-R^8$,
23. $-CH=CH-(CH_2)_m-O-CO-R^7$,
24. $-(CH_2)_m-CH(CH_3)-CO-R^8$,
25. $-(CH_2)_o-CO-R^8$,
26.

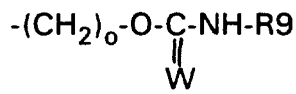

27.

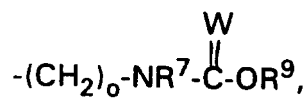

28. $-(CH_2)_o-NR^7-CO-NHR^9$,
29. $-(CH_2)_o-NR^7-SO_2R^9$,
30.

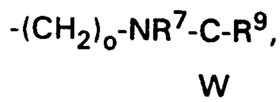

31. $-(CH_2)_nF$,
32. $-(CH_2)_n-O-NO_2$,
33. $-CH_2-N_3$,
34. $-(CH_2)_n-NO_2$,
35. $-CH=N-NR^5R^7$
36. Phthalimido-$(CH_2)_n$-,
37.

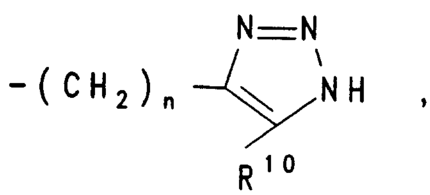

38.

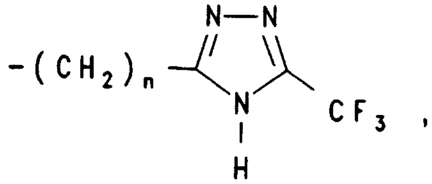

39.

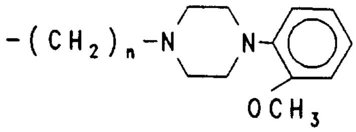

,

40.

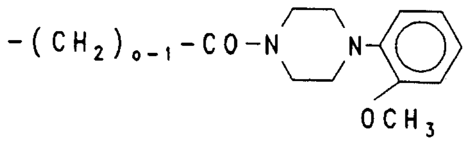

,

41. Phenyl-$SO_2$-NH-N = CH-,
42.

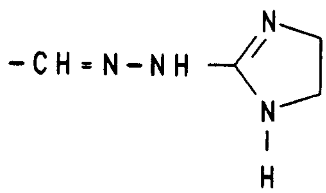

,

43. -$(CH_2)_n$-$SO_2$-$NR^7$-CS-$NR^6R^9$,
44. -$(CH_2)_nSO_2$-$NR^7$-CO-$NR^6R^9$,
45. -$(CH_2)_o$-$SO_2R^9$
46. einem wie unter c) 8. oder 9. definiertem Rest, der am Phenyl mit 1 oder 2 gleichen oder verschiedenen Resten aus der Reihe Halogen, Hydroxy, Methoxy, Trifluormethyl, $CO_2R^3$ und Phenyl substituiert ist,
47. einem wie unter c) 10., 11. oder 19. definierten Rest, worin ein bis alle H-Atome durch Fluor substituiert sind,
48. den unter c) 14. definierten Rest, der mit 1 oder 2 gleichen oder verschiedenen Resten aus der Reihe Methoxycarbonyl und ($C_1$-$C_4$)-Alkyl substituiert ist,
49. -$(CH_2)_nSO_2$-$NR^{7-}$CO-$R^6$,
50. -$(CH_2)_n$-$SO_2$-$NR^7$CS-$R^6$,

d) $R^3$
1. Wasserstoff,
2. ($C_1$-$C_8$)-Alkyl,
3. ($C_3$-$C_8$)-Cycloalkyl,
4. Phenyl,
5. Benzyl,
6. den unter d) 2. definiertem Rest, worin 1 bis alle H-Atome durch Fluor substituiert sind;

e) $R^4$
1. Wasserstoff,
2. ($C_1$-$C_6$)-Alkyl,
3. ($C_3$-$C_8$)-Cycloalkyl,
4. ($C_2$-$C_4$)-Alkenyl oder
5. ($C_2$-$C_4$)-Alkinyl;

f) $R^5$
1. Wasserstoff,
2. ($C_1$-$C_6$)-Alkyl,
3. ($C_3$-$C_8$)-Cycloalkyl,
4. Phenyl oder
5. Benzyl;

g) $R^6$, $R^9$ gleich oder verschieden
1. Wasserstoff,

2. $(C_1-C_6)$-Alkyl, das mit 1-3 Resten aus der Reihe $(C_1-C_6)$-Alkoxy, das seinerseits mit 1-3 Resten aus der Reihe Hydroxy, $(C_1-C_6)$-Alkoxy, Amino, Mono-$(C_1-C_6)$-Alkylamino, Di-$(C_1-C_6)$-Alkylamino substituiert sein kann, $(C_2-C_{10})$-Alkenyl, Hydroxy, Amino, Mono-$(C_1-C_6)$Alkylamino, Di-$(C_1-C_6)$-Alkylamino, $(C_1-C_6)$Alkoxycarbonylamino, $(C_6-C_{12})$-Aryl-$(C_1-C_4)$Alkoxycarbonylamino; $(C_6-C_{10})$-Aryl, $(c_6-C_{10})$Aryl-$(C_1-C_3)$-Alkyl, $(C_1-C_9)$-Heteroaryl, Carboxy und $(C_1-C_4)$-Alkoxycarbonyl substituiert sein kann;
3. $(C_3-C_8)$-Cycloalkyl, wobei der Cycloalkylteil noch durch 1-3 Reste aus der Reihe $(C_1-C_4)$-Alkyl und $(C_2-C_4)$-Alkenyl substituiert sein kann;
4. $(C_3-C_8)$-Cycloalkyl-$(C_1-C_3)$-alkyl,
5. $(C_6-C_{12})$-Aryl, vorzugweise Phenyl,
6. $(C_6-C_{10})$-Aryl-$(C_1-C_4)$-Alkyl,
7. $(C_1-C_9)$-Heteroaryl, welches teilweise oder vollständig hydriert sein kann,
8. einen wie unter g) 5., 6., 7., 9., 15., 16., 17., 19., 20. oder 21. definiertem Rest, substituiert mit 1 oder 2 gleichen oder verschiedenen Resten aus der Reihe Halogen, Hydroxy, $(C_1-C_4)$-Alkyl, Methoxy, Nitro, Cyano, $CO_2R^3$, Trifluormethyl, $NR^{11}R^{12}$ und

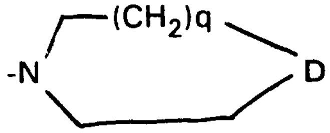

9. $(C_1-C_9)$-Heteroaryl-$(C_1-C_3)$-alkyl, wobei der Heteroarylteil teilweise oder vollständig hydriert sein kann,
10. $(C_1-C_6)$-Alkyl, worin 1 bis alle H-Atome durch Fluor substituiert sind,
11. $(C_2-C_{10})$-Alkenyl, $(C_2-C_{10})$-Alkenoyl oder $(C_2-C_{10})$-Alkadienyl,
12. $(C_3-C_8)$-Cycloalkenyl,
13. $(C_3-C_8)$-Cycloalkenyl-$(C_1-C_3)$-Alkyl,
14. Bi - oder tricyclisches $(C_4-C_{10})$-Cycloalkenyl-$(C_1-C_4)$alkyl, das noch mit 1-3 $(C_1-C_4)$-Alkylreste substituiert sein kann;
15. $(C_6-C_{10})$-Aryl-$(C_1-C_4)$-Alkyl,
16. $(C_6-C_{10})$-Aryl-$(C_3-C_6)$-Alkenyl,
17. $(C_1-C_9)$-Hetaryl-$(C_3-C_6)$-Alkenyl,
18. $(C_3-C_6)$-Alkinyl,
19. $(C_6-C_{10})$-Aryl-$(C_3-C_6)$-Alkinyl,
20. $(C_1-C_9)$-Hetaryl-$(C_3-C_6)$-Alkinyl,
21. $R^6$, $R^9$ gemeinsam mit dem sie tragenden N-Atom ein Hetaryl, das auch teilweise oder vollständig hydriert sein kann;

h) $R^7$
1. Wasserstoff,
2. $(C_1-C_6)$-Alkyl,
3. $(C_3-C_8)$-Cycloalkyl,
4. $(C_6-C_{12})$-Aryl-$(C_1-C_6)$-alkyl, vorzugsweise Benzyl,
5. Phenyl oder
6. $(C_1-C_9)$-Heteroaryl;

i) $R^8$
1. Wasserstoff,
2. $(C_1C_6)$-Alkyl,
3. $(C_3-C_8)$-Cycloalkyl,
4. Phenyl-$(CH_2)_q$-,
5. $OR^6$,
6. $NR^{11}R^{12}$ oder
7.

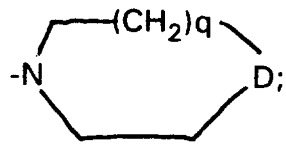

j) $R^{10}$ Cyano, Nitro oder $CO_2R^7$;
k) $R^{11}$ und $R^{12}$ gleich oder verschieden sind und
1. Wasserstoff,
2. $(C_1-C_4)$-Alkyl,
3. Phenyl,
4. Benzyl, oder
5. α-Methylbenzyl;
l) D $NR^{13}$, O oder $CH_2$;
m) $R^{13}$ Wasserstoff, $(C_1-C_4)$Alkyl oder Phenyl;
n) A Biphenylrest, der mit bis zu 4, vorzugsweise bis zu 2 gleichen oder verschiedenen Resten $R^{14}$ oder $R^{15}$ substituiert sein kann;
o) $R^{14}$
1. Halogen,
2. Nitroso,
3. Nitro,
4. Amino,
5. Cyano,
6. Hydroxy,
7. $(C_1-C_6)$-Alkyl,
8. $(C_1-C_4)$-Alkanoyl,
9. $(C_1-C_4)$-Alkanoyloxy,
10. $CO_2R^3$,
11. Methansulfonylamino,
12. Trifluormethansulfonylamino,
13. $-CO-NH-OR^9$,
14. $-SO_2-NR^6R^7$,
15. $-CH_2-OR^7$,
16. $(C_1-C_9)$-Heteroaryl-$(CH_2)_q$-, vorzugsweise 1-Tetrazolyl,
17. $(C_7-C_{13})$-Aroyl,
18.

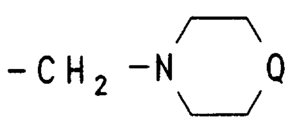

19.

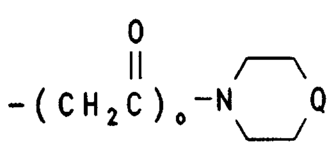

oder
20. $(C_6-C_{12})$-Aryl;
p) $R^{15}$
1. Wasserstoff
2. $(C_1-C_6)$-Alkyl,
3. $(C_3-C_8)$-Cycloalkyl,
4. $(C_6-C_{12})$-Aryl,

5. ($C_7$-$C_{13}$)-Aroyl,
6. ($C_1$-$C_4$)-Alkoxy,
7. ($C_1$-$C_4$)-Alkanoyloxy,
8. ($C_1$-$C_9$)-Heteroaryl,
9. $CO_2R^3$,
10. Halogen,
11. Cyano,
12. Nitro,
13. $NR^6R^7$,
14. Hydroxy,
15. $-CO-NH-CHR^5-CO_2R^3$,
16. Sulfo,
17. $-SO_3R^3$,
18. $-SO_2-NR^7-CO-NR^6R^9$ oder $-SO_2-NR^7-CS-NR^6R^9$,
19. $-NR^7-CO-NR^6-SO_2-CH_2-R^5$,
20. $-C(CF_3)_2OH$,
21. Phosphonooxy,
22. $-PO_3H_2$,
23. $-NH-PO(OH)_2$,
24. $-S(O)_rR^6$,
25. $-CO-R^8$,
26. $-CO-NR^6R^9$,
27. $-CR^{20}(OH)-PO(OH)_2$,
28. den unter o) 20. definiertem Rest,
29.

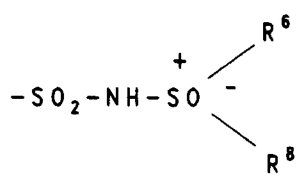 ,

30.

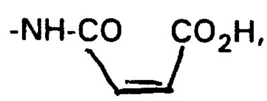

31.

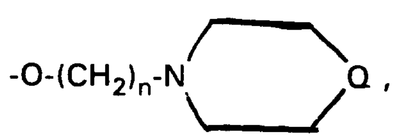

32. 5-Tetrazolyl-NH-CO-,
33. $-CO-NH-NH-SO_2-CF_3$,
34.

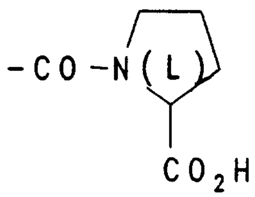

,

35.

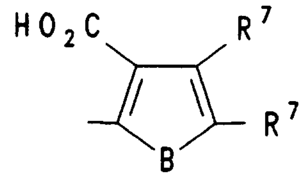

,

36.

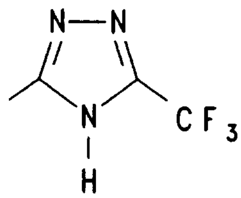

,

37.

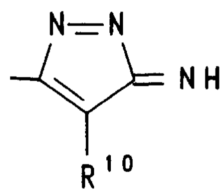

,

38.

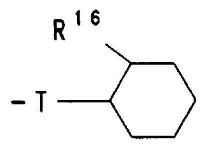

,

39.

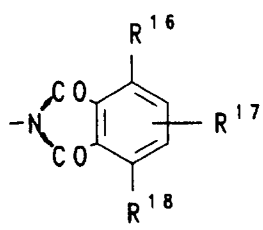

,

40. $-CO-NH-SO_2-R^{19}$

41. $-SO_2-NH-CO-R^6$ oder
42. den unter p) 4. definiertem Rest, substituiert mit 1 oder 2 gleichen oder verschiedenen Resten aus der Reihe Halogen, Cyano, Nitro, $NR^6R^7$ und Hydroxy;
43. $R^{15}$ zusammen mit $R^{14}$ bedeutet $-CO-NH-SO_2-$,
44. $-SO_2-NH-CO-O-R^6$,
45. $-SO_2-NH-SO_2-NR^6R^9$,
46. $-SO_2-NH-SO_2-R^6$,

q) B O, $NR^7$ oder S;
r) W O oder S;
s) L $(C_1-C_3)$-Alkandiyl;
t) $R^{16}$ $CO_2R^3$ oder $CH_2CO_2R^3$;
u) $R^{17}$ Wasserstoff, Halogen, $(C_1-C_4)$-Alkyl oder $(C_1-C_4)$-Alkoxy;
v) $R^{18}$ Wasserstoff, $(C_1-C_4)$-Alkyl oder Phenyl;
w) $R^{19}$

1. $(C_1-C_6)$-Alkyl,
2. $(C_3-C_8)$-Cycloalkyl,
3. Phenyl
4. Benzyl oder
5. den unter w) 1. definiertem Rest, worin ein bis alle H-Atome durch Fluor substituiert sind.

x) T

1. eine Einfachbindung,
2. -CO-,
3. $-CH_2-$,
4. -O-,
5. -S-,
6. $-NR^{21}-$,
7. $-CO-NR^{21}$,
8. $NR^{21}-CO-$,
9. $-O-CH_2-$,
10. $-CH_2-O-$,
11. $-S-CH_2-$,
12. $-CH_2-S$,
13. $-NH-CR^{20}R^{22}$,
14. $-NR^{21}-SO_2$,
15. $SO_2-NR^{21}-$,
16. $-CR^{20}R^{22}-NH$,
17. -CH = CH-,
18. -CF = CF-,
19. -CH = CF-,
20. -CF = CH-,
21. $-CH_2-CH_2-$,
22. $-CF_2-CF_2-$,
23. $-CH(OR^3)-$,
24. $-CH(OCOR^5)-$,
25.

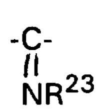

oder
26.

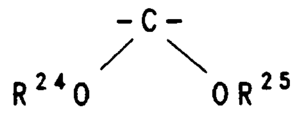 ;

y) $R^{20}$ und $R^{22}$ gleich oder verschieden Wasserstoff, $(C_1\text{-}C_5)$-Alkyl, Phenyl, Allyl oder Benzyl;

z) $R^{21}$ Wassserstoff, $(C_1\text{-}C_6)$Alkyl, Benzyl oder Allyl;

a') $R^{23}$

1. $NR^{20}R^{21}$
2. Ureido,
3. Thioureido,
4. Toluol-4-sulfonyl oder
5. Benzolsulfonyoamino

b') $R^{24}$ und $R^{25}$ gleich oder verschieden $(C_1\text{-}C_4)$-Alkyl oder gemeinsam $-(CH_2)_q-$,

c') Q $CH_2$, NH, O oder S;

d') m eine ganze Zahl von 0 bis 5;

e') n eine ganze Zahl von 1 bis 5;

f') o eine ganze Zahl von 1 bis 10;

g') q 0 oder 1;

h') r 0, 1 oder 2 ist, oder

i') v eine ganze Zahl von 1 bis 6;

sowie deren physiologisch verträglichen Salze.

**3.** Verwendung von Angiotensin-II-Antagonisten gemäß Anspruch 2, dadurch gekennzeichnet, daß eine Verbindung der Formel (I) eingesetzt wird, worin

X N, Y $CR^2$ und Z $CR^2$;
X $CR^2$, Y N und Z $CR^2$;
X $CR^2$, Y $CR^2$ und Z N oder
X, Y und Z jeweils N,

a) $R^1$

1. $(C_1\text{-}C_{10})$-Alkyl,
2. $(C_3\text{-}C_{10})$-Alkenyl,
3. $(C_3\text{-}C_{10})$-Alkinyl,
4. $(C_3\text{-}C_8)$-Cycloalkyl,
5. Benzyl oder
6. Benzyl, das wie oben beschrieben (b 13.) substituiert ist;

b) $R^2$

1. Wasserstoff,
2. Halogen,
3. Nitro,
4. $C_vF_{2v+1}$,
5. Pentafluorphenyl,
6. Cyano,
7. $-O-R^6$,
8. Phenyl,
9. Phenyl-$(C_1\text{-}C_3)$-alkyl,
10. $(C_1\text{-}C_{10})$-Alkyl,
11. $(C_3\text{-}C_{10})$-Alkenyl,
12. Phenyl-$(C_2\text{-}C_6)$-alkenyl,
13. 1-Imidazolyl-$(CH_2)_m$-,
14. 1,2,3-Triazolyl-$(CH_2)_o$-,
15. Tetrazolyl-$(CH_2)_m$-,
16. $-(CH_2)_o$-1-$CHR^7$-$OR^5$,
17. $-(CH_2)_o$-O-$COR^3$,
18. $-COR^8$,
19. $-(CH_2)_o$-CO-$R^8$,

20. $-S(O)_rR^{19}$,
21. $-CH=CH-(CH_2)_m-CHR^3-OR^6$,
22. $-CH=CH-(CH_2)_m-CO-R^8$,
23. $-(CH_2)_o-NH-CO-OR^9$,
24. $-(CH_2)_o-NH-SO_2-R^9$,
25. $-(CH_2)_nF$,
26. $-(CH_2)_o-SO_3R^9$,
27. $-(CH_2)_n-SO_2-NH-CO-NR^6R^9$,
28. $-(CH_2)_n-SO_2-NH-CS-NR^6R^9$, oder
29. einem wie unter b) 8., 9., 10., 11. oder 14. definierten Rest, der wie oben unter c) 46., 47. oder 48. jeweils wie für einen solchen Rest beschrieben substituiert ist,
30. $-(CH_2)_n-SO_2-NR^7-CO-R^6$,
31. $-(CH_2)_n-SO_2-NR^7-CS-R^6$;

c) $R^8$ Wasserstoff; $(C_1-C_5)$-Alkyl, $OR^6$, $NR^{11}R^{12}$ oder Morpholino;
d) T
1. eine Einfachbindung,
2. -CO-,
3. $-CONR^{21}-$,
4. $-CH_2-CH_2-$,
5. $-NR^{21}-CO-$,
6. $-O-CH_2-$,
7. $-CH_2-O-$,
8. $-S-CH_2-$,
9. $-CH_2-S-$,
10. $-NH-CH_2-$,
11. $-CH_2-NH-$ oder
12. $-CH=CH-$ bedeuten,

und die übrigen Reste und Variablen wie in Anspruch 2 definiert sind.

**4.** Verwendung von Angiotensin-II-Antagonisten gemäß Anspruch 2, dadurch gekennzeichnet, daß Verbindungen der Formel (I) eingesetzt werden, wobei
X N, Y $CR^2$ und Z $CR^2$;
X $CR^2$, Y N und Z $CR^2$;
X $CR^2$, Y $CR^2$ und Z N oder
X, Y und Z jeweils N,

a) $R^1$ $(C_1-C_7)$-Alkyl, $(C_3-C_{10})$-Alkenyl oder $(C_3-C_7)$-Alkinyl;
b) $R^2$
1. Chlor,
2. Brom,
3. $C_vF_{2v+1}$ mit v = 1, 2 oder 3,
4. Pentafluorphenyl,
5. $O-R^6$,
6. $-S(O)_rR^{19}$,
7. $(CH_2)_o-1-CHR^7-OR^5$,
8. $(CH_2)_o-O-CO-R^3$,
9. $-COR^8$,
10. $-(CH_2)_o-CO-R^8$,
11. $-CH_2-NH-CO-R^8$,
12. $-(CH_2)_o-NH-SO_2-R^9$,
13. $-CH=CH-CHR^3-OR^6$,
14. Tetrazolyl-$(CH_2)_m$-,
15. $-(CH_2)_nSO_2-NH-CO-NR^6R^9$,
16. $-(CH_2)_o-SO_3R^9$ oder

gegebenenfalls durch Hydroxy substituiertes $(C_1-C_6)$-Alkyl, vorzugsweise Hydroxymethyl;
c) $R^3$ Wasserstoff, $(C_1-C_4)$-Alkyl oder Benzyl;
d) $R^6$, $R^9$ gleich oder verschieden
1. Wasserstoff,

2. $(C_1-C_6)$-Alkyl, das mit 1 bis 3 Resten aus der Reihe $(C_1-C_6)$-Alkoxy, das seinerseits mit 1 bis 3 Resten aus der Reihe Hydroxy, $(C_1-C_6)$-Alkoxy, Amino, Mono-$(C_1-C_6)$-Alkylamino, Di-$(C_1-C_6)$-Alkylamino substituiert sein kann, $(C_2-C_{10})$-Alkenyl, Hydroxy, Amino, Mono-$(C_1-C_6)$Alkylamono, Di-$(C_1-C_6)$-Alkylamino, $(C_1-C_6)$Alkoxycarbonylamino, $(C_6-C_{12})$-Aryl-$(C_1-C_4)$Alkoxycarbonylamino; $(C_6-C_{10})$-Aryl, $(C_6-C_{10})$Aryl-$(C_1-C_3)$-Alkyl, $(C_1-C_9)$-Heteroaryl, Carboxy und $(C_1-C_4)$-Alkoxycarbonyl substituiert sein kann;
3. $(C_3-C_6)$-Cycloalkyl,
4. $(C_3-C_6)$-Cycloalkyl-$(C_1-C_3)$-alkyl,
5. Phenyl,
6. Phenyl-$(C_1-C_3)$-Alkyl,
7. $(C_1-C_7)$-Heteroaryl, weiches teilweise oder vollständig hydriert sein kann,
8. einen wie oben unter g) 5., 6., 7. oder 9., 14. bis 16. und 18. bis 20. definierten Rest, substituiert mit 1 oder 2 gleichen oder verschiedenen Resten aus der Reihe Halogen, Hydroxy, $(C_1-C_4)$-Alkyl, Methoxy, Nitro, Cyano,
$CO_2R^3$, Trifluormethyl, $-NR^{11}R^{12}$ und

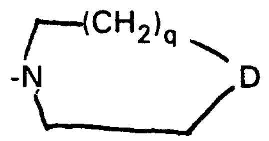

,

9. $(C_1-C_9)$-Heteroaryl-$(C_1-C_3)$-alkyl, wobei der Heteroarylteil teilweise oder vollständig hydriert sein kann,
10. $(C_1-C_6)$-Alkyl, worin 1 bis alle H-Atome durch Fluor substituiert sind,
11. $(C_2-C_4)$-Alkenyl oder $(C_3-C_6)$-Alkenoyl,
12. $(C_3-C_6)$-Cycloalkenyl,
13. $(C_3-C_6)$-Cycloalkenyl-$(C_1-C_3)$-Alkyl,
14. Bi- oder tricyclisches $(C_4-C_{10})$-Cycloalkenyl-$(C_1-C_{10})$-alkyl, das noch mit 1 bis 3 $(C_1-C_4)$-Alkylresten substituiert sein kann;
15. $(C_6-C_{10})$-Aryl-$(C_1-C_3)$-Alkyl,
16. $(C_6-C_{10})$-Aryl-$(C_3-C_6)$-Alkenyl,
17. $(C_1-C_6)$-Hetaryl-$(C_3-C_6)$-Alkenyl,
18. $(C_3-C_6)$-Alkinyl,
19. $(C_6-C_{10})$-Aryl-$(C_3-C_6)$-Alkinyl,
20. $(C_1-C_6)$-Hetaryl-$(C_3-C_6)$-Alkinyl,
21. $R^6$, $R^9$ gemeinsam mit dem sie tragenden N-Atom ein Hetaryl, das auch teilweise oder vollständig hydriert sein kann;

e) $R^7$ Wasserstoff, $(C_1-C_4)$-Alkyl, $(C_1-C_9)$-Heteroaryl, oder $(C_6-c_{12})$-Aryl-$(C_1-C_4)$-Alkyl;

f) $R^{14}$
1. $(C_1-C_4)$-Alkyl,
2. $(C_1-C_4)$-Alkoxy,
3. Cyano,
4. Amino,
5. Nitroso,
6. Nitro,
7. Fluor,
8. Chlor,
9. Brom,
10. $(C_1-C_9)$-Heteroaryl-$CH_2$-,
11. $(C_1-C_4)$-Alkanoyloxy,
12. $(C_1-C_4)$Alkanoyl,
13. Benzoyl,
14. $-NH-CO-R^7$ oder
15. Tetrazolyl;

h) $R^{15}$
1. $(C_1-C_4)$-Alkyl,
2. $(C_6-C_{12})$-Aryl,

3. ($C_1$-$C_3$)-Alkanoyloxy,
4. ($C_1$-$C_4$)-Alkoxy,
5. ($C_1$-$C_9$)-Heteroaryl, vorzugsweise 5-Tetrazolyl,
6. Cyano,
7. Nitro,
8. Hydroxy,
9. $-S(O)_rR^6$,
10. $-SO_3R^3$,
11. Chlor,
12. Brom,
13. Benzoyl,
14. $-CO_2R^3$,
15. $-CO-NH-R^6$,
16. $-CO-R^8$,
17. $-SO_2-NR^6R^7$,
18. $-SO_2-NH-CO-NR^6R^9$,
19. $-PO_3H_2$,
20. $-CO-CHR^5-CO_2H$,
21. $-NH-CO-NH-SO_2-CH_2-R^5$,
22. 5-Tetrazolyl-NH-CO-,
23.

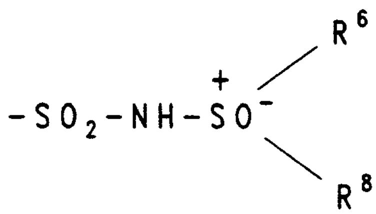

,

24.

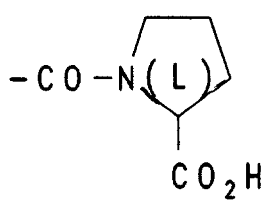

,

25.

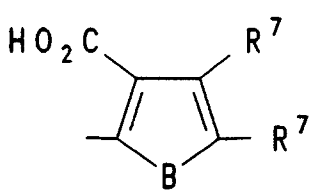

,

26.

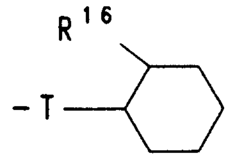,

27.

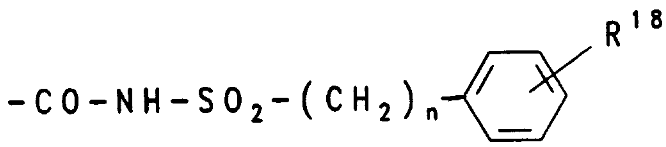

oder

28. den unter h) 2) definierten Rest, substituiert wie oben definiert (siehe p) 42),

29. $R^{15}$ mit $R^{14}$ zusammen $-CO-NH-SO_2-$`

30. $-SO_2-NH-COO-R^6-$

31. $-SO_2-NH-SO_2-NR^6R^9$,

32. $-SO_2-NH-SO_2-R^6$;

i) $R^{18}$ Wasserstoff, Methyl oder Ethyl;

j) T eine Einfachbindung, -O-, -CO-, -NHCO- oder $-OCH_2-$;

k) q = O und L = Methylen, bedeuten

und die übrigen Reste und Variablen wie in Anspruch 2 definiert sind.

**5.** Verwendung von Angiotensin-II-Antagonisten gemaß Anspruch2, dadurch gekennzeichnet, daß Verbindungen der Formel (I) eingesetzt werden, wobei

Z Stickstoff,

X, Y unabhängig voneinander $CR^2$,

$R^1$ $(C_2-C_7)$-Alkyl, $(C_3-C_7)$-Alkenyl oder $(C_3-C_7)$-Alkinyl,

$R^2$ Wasserstoff, Halogen, Nitro, $(C_1-C_3)$-Perfluoralkyl, Cyano, $(C_1-C_{10})$-Alkyl, $(C_3-C_{10})$-Alkenyl, $-CH_2OR^5$` $-S(O)_r-R^{19}$, $-CO-R^8$ oder $-O-R^6$,

$R^5$ Wasserstoff oder $(C_1-C_6)$-Alkyl

$R^6$, $R^9$

1. Wasserstoff,
2. $(C_1-C_6)$-Alkyl, das mit 1 bis 3 Resten aus der Reihe $(C_1-C_6)$-Alkoxy, das seinerseits mit 1 bis 3 Resten aus der Reihe Hydroxy, $(C_1-C_6)$-Alkoxy, Amino, Mono-$(C_1-C_6)$-Alkylamino, Di-$(C_1-C_6)$-Alkylamino substituiert sein kann, $(C_2-C_{10})$-Alkenyl, Hydroxy, Amino, Mono-$(C_1-C_6)$-Alkylamono, Di-$(C_1-C_6)$-Alkylamino, $(C_1-C_6)$Alkoxycarbonylamino, $(C_6-C_{10})$-Aryl, $(C_6-C_{10})$Aryl-$(C_1-C_3)$-Alkyl, $(C_1-C_9)$-Heteroaryl, Carboxy und $(C_1-C_4)$-Alkoxycarbonyl substituiert sein kann;
3. $(C_3-C_8)$-Cycloalkyl,
4. $(C_3-C_6)$-Ccloalkyl-$(C_1-C_3)$-alkyl,
5. $(C_6-C_{12})$-Aryl, vorzugsweise Phenyl,
6. $(C_6-C_{10})$-Aryl-$(C_1-C_4)$-Alkyl,
7. $(C_1-C_9)$-Heteroaryl, welches teilweise oder vollständig hydriert sein kann,
8. $(C_1-C_9)$-Heteroaryl-$(C_1-C_3)$-alkyl, wobei der Heteroarylteil teilweise oder vollständig hydriert sein kann,
9. einen wie vorstehend unter 5., 6., 7. und 8. definierten Rest, substituiert mit 1 oder 2 gleichen oder verschiedenen Resten aus der Reihe Halogen, Hydroxy, $(C_1-C_4)$-Alkyl, Methoxy, Nitro, Cyano, $CO_2R^3$, Trifluormethyl, $-NR^{11}R^{12}$ und

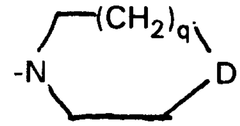

10. $(C_1-C_6)$-Alkyl, worin 1 bis alle H-Atome durch Fluor substituiert sind,
11. $(C_2-C_6)$-Alkenyl oder $(C_3-C_6)$-Alkenoyl,
12. $(C_3-C_8)$-Cycloalkenyl,
13. $(C_3-C_8)$-Cycloalkenyl-$(C_1-C_3)$-Alkyl,
14. $(C_6-C_{10})$-Aryl-$(C_1-C_4)$-Alkyl,
15. $(C_6-C_{10})$-Aryl-$(C_3-C_6)$-Alkenyl,
16. $(C_1-C_9)$-Hetaryl-$(C_3-C_6)$-Alkenyl,
17. $(C_3-C_6)$-Alkinyl,
18. $(C_6-C_{10})$-Aryl-$(C_3-C_6)$-Alkinyl
19. $(C_1-C_9)$-Hetaryl-$(C_3-C_6)$-Alkinyl,
20. $R^6$, $R^9$ gemeinsam mit dem sie tragenden N-Atom ein Hetaryl, das auch teilweise oder vollständig hydriert sein kann,

| | |
|---|---|
| $R^7$ | Wasserstoff, |
| $R^8$ | Wasserstoff oder $-OR^6$, |
| $R^{11}$, $R^{12}$ | unabhängig voneinander Wasserstoff oder $(C_1-C_4$-Alkyl |
| D | $-NR^{13}$, -O oder $-CH_2$, |
| $R^{13}$ | Wasserstoff oder $(C_1-C_4)$-Alkyl, |
| A | Biphenylrest der mit einem Rest $R^{15}$ oder mit $R^{14}$ und $R^{15}$ zusammen substituiert ist, |
| $R^{15}$ | $-SO_2-NR^7-CO-NR^6R^9$, $-SO_2-NH-COO-R^6$, $-SO_2-NH-SO_2-NR^6-R^9$, $-SO_2-NH-CO-R^6$ oder $-SO_2-NH-SO_2-R^6$; oder |
| $R^{14}$ und $R^{15}$ | zusammen $-CO-NH-SO_2-$ sein kann, |
| L | $-CH_2-$, |
| q | Null und |
| r | Null, 1 oder 2, bedeuten |

sowie deren physiologisch verträglichen Salze.

**6.** Verwendung von Angiotensin-II-Antagonisten gemäß Anspruch 1, dadurch gekennzeichnet, daß Verbindungen der Formel (III) eingesetzt werden,

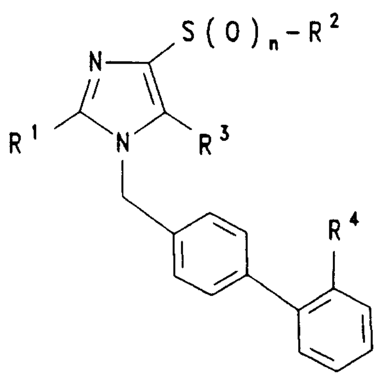

(III)

wobei:

a) $R^1$ $(C_1-C_7)$-Alkyl, $(C_3-C_7)$-Alkenyl oder $(C_3-C_7)$-Alkinyl;
b) $R^2$ $(C_1-C_6)$-Alkyl, vorzugsweise Methyl
c) $R^3$ $-CO-R^6$
d) $R^4$ $SO_2-NH-CO-NR^7R^9$,
$SO_2-NH-COO-R^7$,
$SO_2-NH-CO-R^7$ oder

$SO_2$-N = CH-N($CH_3$)$_2$
e) $R^6$ Wasserstoff oder
$OR^7$,
f) $R^7$, $R^9$ gleich oder verschieden Wasserstoff, ($C_1$-$C_6$)-Alkyl, vorzugsweise Methyl, Ethyl oder Propyl, ($C_3$-$C_8$)-Cycloalkyl, ($C_3$-$C_6$)-Cycloalkyl-($C_1$-$C_3$)-alkyl, ($C_6$-$C_{12}$)-Aryl, vorzugsweise Phenyl oder ($C_6$-$C_{10}$)Aryl-($C_1$-$C_4$)-alkyl, ($C_2$-$C_6$)-Alkenyl, ($C_3$-$C_6$)Alkenoyl oder ($C_3$-$C_6$)-Alkinyl
g) n O, 1 oder 2; bedeuten
sowie deren verträgliche Salze.

**7.** Verwendung von Angiotensin-II-Antagonisten gemäß Anspruch 1, dadurch gekennzeichnet, daß Verbindungen der Formel (II) eingesetzt werden

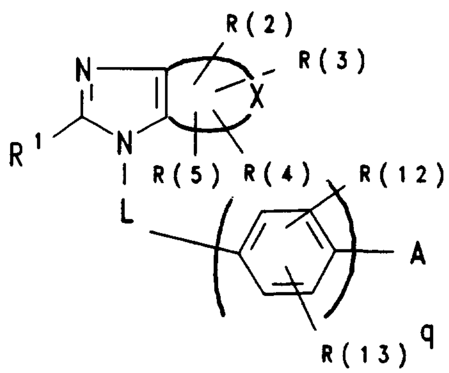

(II)

wobei:

X — steht für einen monocylischen Rest mit 3, 4 oder 5 Ringatomen oder einen bicyclischen Rest mit 8 - 10 Ringatomen, der ganz oder teilweise hydriert sein kann und in dem eine oder mehrere CH- bzw. $CH_2$-Gruppen durch N, NH oder O ersetzt sein können;

R(1)

1. ($C_1$-$C_{10}$)-Alkyl,
2. ($C_3$-$C_{10}$)-Alkenyl,
3. ($C_3$-$C_{10}$)-Alkinyl,
4. OR(6),
5. ($C_3$-$C_8$)-Cycloalkyl,
6. ($C_4$-$C_{10}$)-Cycloalkylalkyl,
7. ($C_5$-$C_{10}$)-Cycloalkylalkenyl,
8. ($C_5$-$C_{10}$)-Cycloalkinyl,
9. $(CH_2)_m$-B-$(CH_2)_n$-R(7),
10. Benzyl,
11. einem wie unter 1., 2., 3. oder 9. definiertem Rest, der monosubstituiert ist mit $CO_2$R(6),
12. einem wie unter 1., 2., 3. oder 9. definiertem Rest, worin 1 bis H-Atome durch Fluor substituiert sind, oder
13. den unter 10. definiertem Rest, der am Phenyl mit 1 oder 2 gleichen oder verschiedenen Resten aus der Reihe Halogen, ($C_1$-$C_4$)-Alkoxy und Nitro substituiert ist;

R(2), R(3), R(4) und R(5) — gleich oder verschieden sind und

1. Wasserstoff, Halogen, Hydroxy, Cyano, Nitro, Sulfo, Formyl, Benzoyl, ($C_1$-$C_6$)-Acyl, ($C_1$-$C_8$)-Acyloxy, Mercapto, Carboxy, ($C_1$-$C_4$)-Alkoxycarbonyl,
2. einen linearen oder verzweigten, gegebenenfalls substituierten, bis zu 6 C-Atome enthaltene Alkyl-, Alkenyl- ,Alkoxy- oder Allylthio-Rest,

3. einen Aryl-, Arylalkyl oder Arylalkenyl-Rest, in denen der Alkyl- und Alkenyl-Substituent unverzweigt oder verzweigt bis zu 6 C-Atomen aufweist und der Aryl-Substituent steht für einen monocyclischen Rest mit 5 oder 6 Ringatomen oder für kondensierte Ringe mit 8 bis 14 Ringatomen, in denen ein oder mehrere Heteroatome wie O, N oder S enthalten sind und die gegebenenfalls substituiert sind,
4. einen Rest

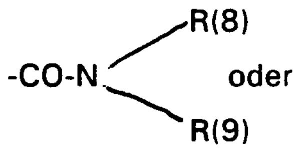 oder 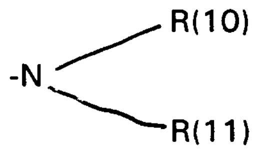

bedeuten;

R(6)
1. Wasserstoff,
2. $(C_1-C_8)$-Alkyl,
3. $(C_3-C_8)$-Cycloalkyl,
4. Phenyl,
5. Benzyl oder
6. den unter 2. definiertem Rest, worin 1 bis alle H-Atome durch Fluor substituiert sind;

R(7)
1. Wasserstoff,
2. $(C_1-C_6)$-Alkyl,
3. $(C_3-C_8)$-Cycloalkyl,
4. $(C_2-C_4)$-Alkenyl oder
5. $(C_2-C_4)$-Alkinyl,

R(8) und R(9) oder R(10) und R(11) entweder gleich oder verschieden für
1. Wasserstoff,
2. $(C_1-C_6)$-Alkyl oder $(C_1-C_6)$-Alkenyl, unsubstituiert oder substituiert druch Halogen, Hydroxy oder $(C_1-C_6)$-Alkoxy,
3. Aryl oder $(C_1-C_6)$-Alkylaryl, worin der Arylrest monocyclisch mit 5 oder 6 Ringatomeen oder bicyclisch mit 8 - 10 Ringatomen ist, gegebenenfalls ein oder mehrere Heteroatome wie O, N und S enthält und mit 1 oder 2 gleichen oder verschiedenen Resten aus der Reihe Halogen, Hydroxy, Nitro, $(C_1-C_6)$-Alkyl, $(C_1-C_6)$-Alkenyl, $(C_1-C_4)$Alkanoyl, $(C_1-C_4)$-Alkanoyloxy und $CO_2R(6)$ substituiert ist;

oder
R(8) und R(9) und R(11) zusammen mit dem sie tragenden N-Atom einen 4- bis 8-gliedrigen Ring bilden, der gesättigt oder ungesättigt ist, ein weiteres Heteroatom ausgewählt aus der Gruppe N, O und S enthalten kann und unsubstituiert oder durch Halogen, Hydroxy-, $(C_1-C_4)$-Alkyl, $(C_1-C_4)$-Alkenyl, $(C_1-C_4)$-Alkyloxy und $CO_2R(6)$ substituiert ist.
oder
R(10) und R(11) entweder gleich oder verschieden stehen für einen Acylrest aus bis zu 6 C-Atomen oder einen $(C_1-C_6)$-Alkyl oder $(C_6-C_{12})$-Arylrest, die gegebenenfalls durch Halogen oder $(C_1-C_6)$-Alkylreste substituiert sind;

L $(C_1-C_3)$-Alkandiyl bedeutet;

R(12) und R(13) gleich oder verschieden sind und
1. Wasserstoff,
2. Halogen,
3. Nitro,

4. $(C_1-C_4)$-Alkyl oder
5. $(C_1-C_2)$-Alkoxy,

bedeuten;

q Null oder 1 ist,

A steht für entweder

1. den Rest eines Heterocyclus mit 5 - 10 Ringatomen, der mono- oder bicyclisch sein kann, und wovon bis zu 9 Ringatome C-Atome sind, der unsubstituiert oder mit bis zu 6, vorzugsweise bis zu 3 gleichen oder verschiedenen Resten R(14) und R(15) substituiert ist,

oder

2. einen Biphenylrest, der unsubstituiert oder mit bis zu 4, vorzugsweise bis zu 2 gleichen oder verschiedenen Resten R(14) und R(15) substituiert ist, wobei A aber zwingend mit mindestens einem unter R(15) 18., 19., 28., 40. oder 42. definierten Rest substituiert und q = Null ist.

R(14)

1. Halogen,
2. Oxo,
3. Nitroso,
4. Nitro,
5. Amino,
6. Cyano,
7. Hydroxy,
8. $(C_1-C_6)$-Alkyl,
9. $(C_1-C_4)$-Alkanoyl,
10. $(C_1-C_4)$-Alkanoyloxy,
11. $CO_2R(6)$,
12. Methansulfonylamino,
13. Trifluormethansulfonylamino,
14. -CO-NH-OR(16),
15. $-SO_2-NR(17)R(18)$,
16. $-CH_2-OR(18)$,
17. $(C_1-C_4)$-Heteroaryl-$(CH_2)_q$-, vorzugsweise 1-Tetrazolyl,
18. $(C_7-C_{13})$-Aroyl,
19.

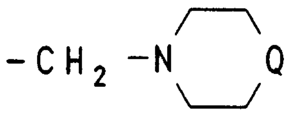

20.

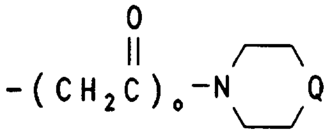

oder

21. $(C_6-C_{12})$-Aryl

bedeutet;

R(15)

1. Wasserstoff,
2. $(C_1-C_6)$-Alkyl,
3. $(C_3-C_8)$-Cycloalkyl,
4. $(C_6-C_{12})$-Aryl,

5. ($C_7$-$C_{13}$)-Aroyl,
6. ($C_1$-$C_4$)-Alkoxy,
7. ($C_1$-$C_4$)-Alkanoyloxy,
8. ($C_1$-$C_9$)-Heteroaryl,
9. $CO_2R(6)$,
10. Halogen,
11. Cyano,
12. Nitro,
13. NR(17)R(18),
14. Hydroxy,
15. -CO-NH-CHR(19)-$CO_2R(6)$,
16. Sulfo,
17. -$SO_3R(6)$,
18. -$SO_2$-NR(18)-CO-NR(17)R(16), -$SO_2$-NR(18)-CO-O-R(17), -$SO_2$N(CO-O-R-(17))$_2$ oder -$SO_2$-NR(18)-CS-NR(17)R(16),
19. -NR(18)-SO-NR(17)-$SO_2$-$CH_2$-R(18),
20. -$C(CF_3)_2OH$,
21. Phosphonooxy,
22. -$PO_3H_2$,
23. -NH-$PO(OH)_2$,
24. -$S(O)_rR(17)$,
25. -CO-R(20),
26. -CO-NR(17)R(16),
27.

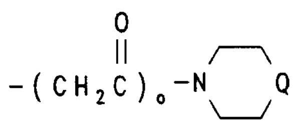

28.

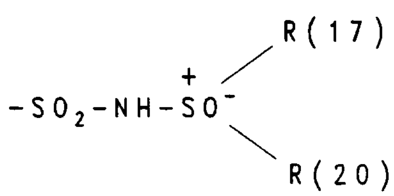

29.

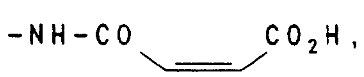

30.

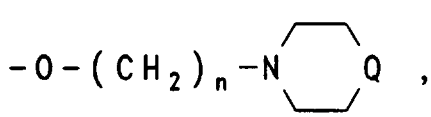

31. 5-Tetrazolyl-NH-CO-,

32. $-CO-NH-NH-SO_2CF_3$,

33.

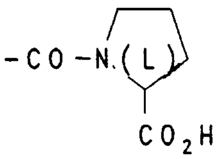

,

34.

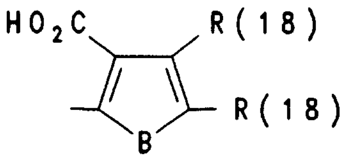

,

35.

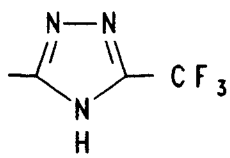

,

36.

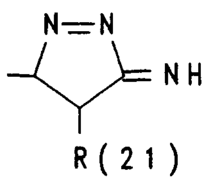

,

37.

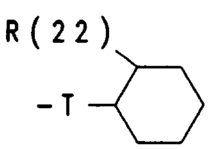

,

38.

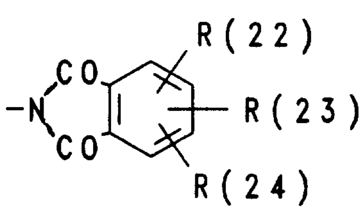

,

39. $-CO-NH-SO_2-R(6)$,
40. $-SO_2-NH-CO-R(17)$,
41. den unter 4. definierten Rest, substituiert mit 1 oder 2 gleichen oder verschiedenen Resten aus der Reihe Halogen, Cyano, Nitro, NR(17)R(18) und Hydroxy,
42. R(15) zusammen mit R(14) bedeutet $-CO-NH-SO_2-$;

R(16), R(17) gleich oder verschieden
1. Wasserstoff,
2. $(C_1-C_6)$-Alkyl,
3. $(C_3-C_8)$-Cycloalkyl,
4. $(C_6-C_{12})$-Aryl, vorzugsweise Phenyl,
5. $(C_6-C_{10})$-Aryl-$(C_1-C_4)$-Alkyl,
6. $(C_1-C_9)$-Heteroaryl, welches teilweise oder vollständig hydriert sein kann, vorzugsweise 2-Pyrimidinyl, 1-Piperidinyl oder Chinuclidinyl,
7. $(C_3-C_6)$-Alkenoyl,
8. einen wie unter 4., 5., 6., 9., 14., 15., 16., 18., 19., oder 20. definierten Rest, substituiert mit 1 oder 2 gleichen oder verschiedenen Resten aus der Reihe Hydroxy, Methoxy, Nitro, Cyano, $CO_2R(6)$, Trifluormethyl, -NR(25)R-(26) und

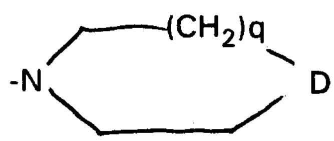

9. $(C_1-C_9)$-Heteroaryl-$(C_1-C_3)$-alkyl, wobei der Heteroarylteil teilweise oder vollständig hydriert sein kann,
10. den unter 2. definierten Rest, woein 1 bis alle H-Atome durch Fluor substituier sind,
11. $(C_2-C_6)$-Alkenyl,
12. $(C_3-C_8)$-Cycloalkenyl,
13. $(C_3-C_8)$-Cycloalkenyl-$(C_1-C_3)$-alkyl,
14. $(C_3-C_8)$-Cycloalkyl-$(C_1-C_4)$-alkyl,
15. $(C_6-C_{10})$-Aryl-$(C_3-C_6)$-Alkenyl,
16. $(C_1-C_9)$-Hetaryl-$(C_3-C_6)$-Alkenyl,
17. $(C_3-C_6)$-Alkinyl,
18. $(C_6-C_{10})$-Aryl-$(C_3-C_6)$-Alkinyl,
19. $(C_1-C_9)$-Hetaryl-$(C_3-C_6)$-Alkinyl,
20. einen Rest der Formel

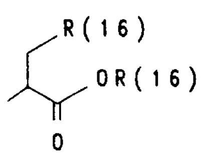

,

wobei R(16) die Bedeutung von 20. nicht haben kann.
21. R(16)R(17) gemeinsam mit dem sie tragenden N-Atom ein Hetaryl bilden, das auch teilweise oder vollständig hydriert sein kann;

R(18)
1. Wasserstoff,
2. $(C_1-C_6)$-Alkyl,
3. $(C_3-C_8)$-Cycloalkyl,
4. $(C_6-C_{12})$-Aryl-$(C_1-C_6)$-alkyl, vorzugsweise Benzyl,
5. Phenyl oder

6. ($C_1$-$C_9$)-Heteroaryl;

R(19)

1. Wasserstoff,
2. ($C_1$-$C_6$)-Alkyl,
3. ($C_3$-$C_8$)-Cycloalkyl,
4. Phenyl oder
5. Benzyl;

R(20)

1. Wasserstoff,
2. ($C_1$-$C_6$)-Alkyl,
3. ($C_3$-$C_8$)-Cycloalkyl,
4. Phenyl-$(CH_2)_q$-,
5. OR(19),
6. NR(25)R(26) oder
7.

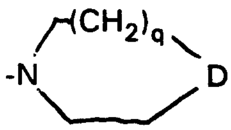

R(21) Cyano, Nitro oder $CO_2R(18)$ bedeutet;
R(22) $CO_2R(6)$ oder $CH_2CO_2R(6)$ bedeutet;
R(23) Wasserstoff, Halogen, ($C_1$-$C_4$)-Alkyl oder ($C_1$-$C_4$)-Alkoxy bedeutet;
R(24) Wasserstoff, ($C_1$-$C_4$)-Alkyl oder Phenyl bedeutet;
R(25) und R(26) gleich oder verschieden sind und

1. Wasserstoff,
2. ($C_1$-$C_4$)-Alkyl,
3. Phenyl,
4. Benzyl oder
5. $\alpha$-Methylbenzyl

bedeuten;

D NR(23), O oder $CH_2$ bedeutet;
B O, NR(18) oder S bedeutet;
T

1. eine Einfachbindung,
2. -CO-,
3. $-CH_2-$,
4. -O-,
5. -S-,
6. -NR(28)-,
7. -CO-NR(28)-,
8. -NR(28)-CO-,
9. $-O-CH_2-$,
10. $-CH_2-O-$,
11. $-S-CH_2-$,
12. $-CH_2-S-$,
13. -NH-CR(27)R(29)-,
14. $-NR(28)-SO_2-$,
15. -CR(27)R(29)-NH-,
16. -CR(27)R(29)-NH-,
17. -CH = CH-,
18. -CF = CF-,
19. -CH = CF-,
20. -CF = CH-,
21. $-CH_2-CH_2-$,
22. $-CF_2-CF_2-$,

23. -CH(OR(6))-,
24. -CH(OCOR(19))-,
25.

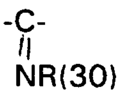

oder
26.

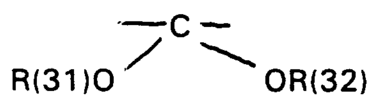

R(27) und R(29) gleich oder verschieden sind und Wasserstoff, ($C_1$-$C_5$)-Alkyl, Phenyl, Allyl oder Benzyl bedeuten;
R(28) Wasserstoff, ($C_1$-$C_6$)-Alkyl, Benzyl oder Allyl bedeutet;
R(30)
1. NR(27)R(28),
2. Ureido,
3. Thioureido,
4. Toluol-4-sulfonyl oder
5. Benzolsulfonylamino

bedeuten;
R(31) und R(32) gleich oder verschieden sind und ($C_1$-$C_4$)-Alkyl bedeuten oder gemeinsam für $-(CH_2)_q-$ stehen;
Q $CH_2$, NH, O oder S bedeutet;
n eine ganze Zahl von 1 bis 5 ist;
m eine ganze Zahl von 0 bis 3 ist;
o eine ganze Zahl von 1 bis 10 ist;
r Null, 1 oder 2 ist,

sowie deren physiologisch verträgliche Salze.

**8.** Verwendung von Angiotensin-II-Antagonisten gemäß Anspruch 1, dadurch gekennzeichnet, daß Verbindungen der Formel (IV) eingesetzt werden

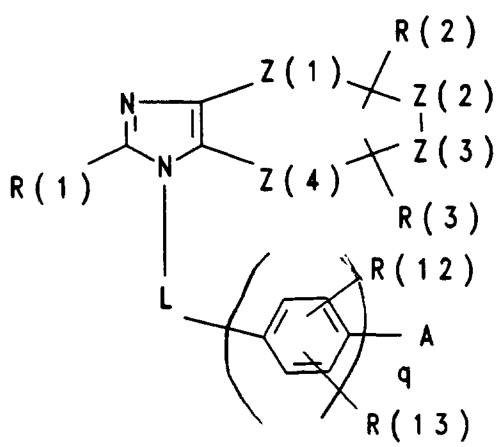

(IV)

wobei:
Z(1), Z(2), Z(3) und Z(4):
1. $-CH_2-$,

2. -CH= ,
3. ein unter 2. definierter Rest, wobei 1 oder 2 Methin-Gruppen durch Stickstoff ersetzt sind; bevorzugt ist Z(4) = N,

R(1)
1. ($C_1C_{10}$)-Alkyl,
2. ($C_3$-$C_{10}$)-Alkenyl,
3. ($C_3$-$C_{10}$)-Alkinyl,
4. ($C_3$-$C_8$)-Cycloalkyl,
5. Benzyl oder
6. Benzyl, das wie oben beschrieben substituiert ist;

R(2) und R(3) gleich oder verschieden stehen für:
1. Wasserstoff,
2. Hydroxy,
3. Halogen,
4. einen linearen oder verzweigten ($C_1$-$C_6$)-Alkylrest, unsubstituiert oder substituiert durch einen oder mehrere gleiche oder verschiedene Substituenten aus der Reihe Halogen, Hydroxy, ($C_1$-$C_4$)-Alkoxy, ($C_1$-$C_4$)-Alkylthio, Mercapto,
5. -$CO_2$R(6);

T eine Einfachbindung, -O-, -CO-, -NHCO- oder -$OCH_2$- bedeutet

und die übrigen Reste und Variablen wie in Anspruch 7 definiert sind.

**9.** Verwendung von Angiotensin II-Antagonisten gemäß Anspruch 8, dadurch gekennzeichnet, daß Verbindungen der Formel (IV), in welcher

R(1) ($C_1$-$C_7$)-Alkyl, ($C_3$-$C_7$)-Alkenyl oder ($C_3$-$C_7$)-Alkinyl bedeutet;
R(6) Wasserstoff oder ($C_1$-$C_4$)-Alkyl bedeutet;
R(12) und R(13) gleich oder verschieden und Wasserstoff und ($C_1$-$C_4$)-Alkyl bedeuten;
R(14)
1. ($C_1$-$C_4$)-Alkyl,
2. ($C_1$-$C_4$)-Alkoxy,
3. Cyano,
4. Amino,
5. Nitro,
6. Fluor, Chlor oder Brom,
7. ($C_1$-$C_4$)-Heteroaryl-$CH_2$,
8. ($C_1$-$C_4$)-Alkanoyloxy,
9. ($C_1$-$C_4$)-Alkanoyl,
10. Benzoyl oder
11. Tetrazolyl bedeutet;

R(15)
1. ($C_1$-$C_4$)-Alkyl,
2. ($C_6$-$C_{12}$)-Aryl,
3. ($C_1$-$C_3$)-Alkanoyloxy,
4. ($C_1$-$C_4$)-Alkoxy,
5. ($C_1$-$C_9$)-Heteroaryl, vorzugsweise 5-Tetrazolyl,
6. Cyano,
7. Nitro,
8. Hydroxy,
9. $SO_3$R(6),
10. Chlor, Brom,
11. $CO_2$R(6),
12. CO-NH-R(19),
13. CO-R(20),
14. $SO_2$-NR(18)-CO-NR(17)R(16),
15. $SO_2$-NR(18)-CO-O-R(17) oder $SO_2$N(CO-OR(17))$_2$,
16. CO-CHR(19)-$CO_2$H,

17. $(C_1-C_4)$-Alkyl-$CO_2H$,
18. NH-CO-NH-$SO_2$-$CH_2$-R(19),
20.

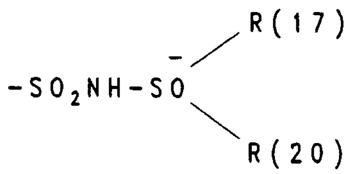

,

21.

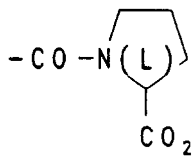

,

22.

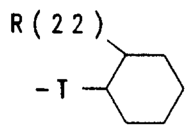

oder
23. R(14) mit R(15) zusammen -CO-NH-$SO_2$;

L -$CH_2$-;
R(18) Wasserstoff;
R(25) und R(26) unabhängig voneinander Wasserstoff oder $(C_1-C_4)$-Alkyl

bedeuten,
sowie deren physiologisch verträglichen Salze eingesetzt werden.

**10.** Verwendung von Angiotensin II-Antagonisten der Formel (I), (II), (III) oder (IV) gemäß einem der Ansprüche 2 bis 9 zur Herstellung eines Arzneimittels zur Behandlung von Kognitiven oder erektilen Dysfunktionen.

Fig. 1

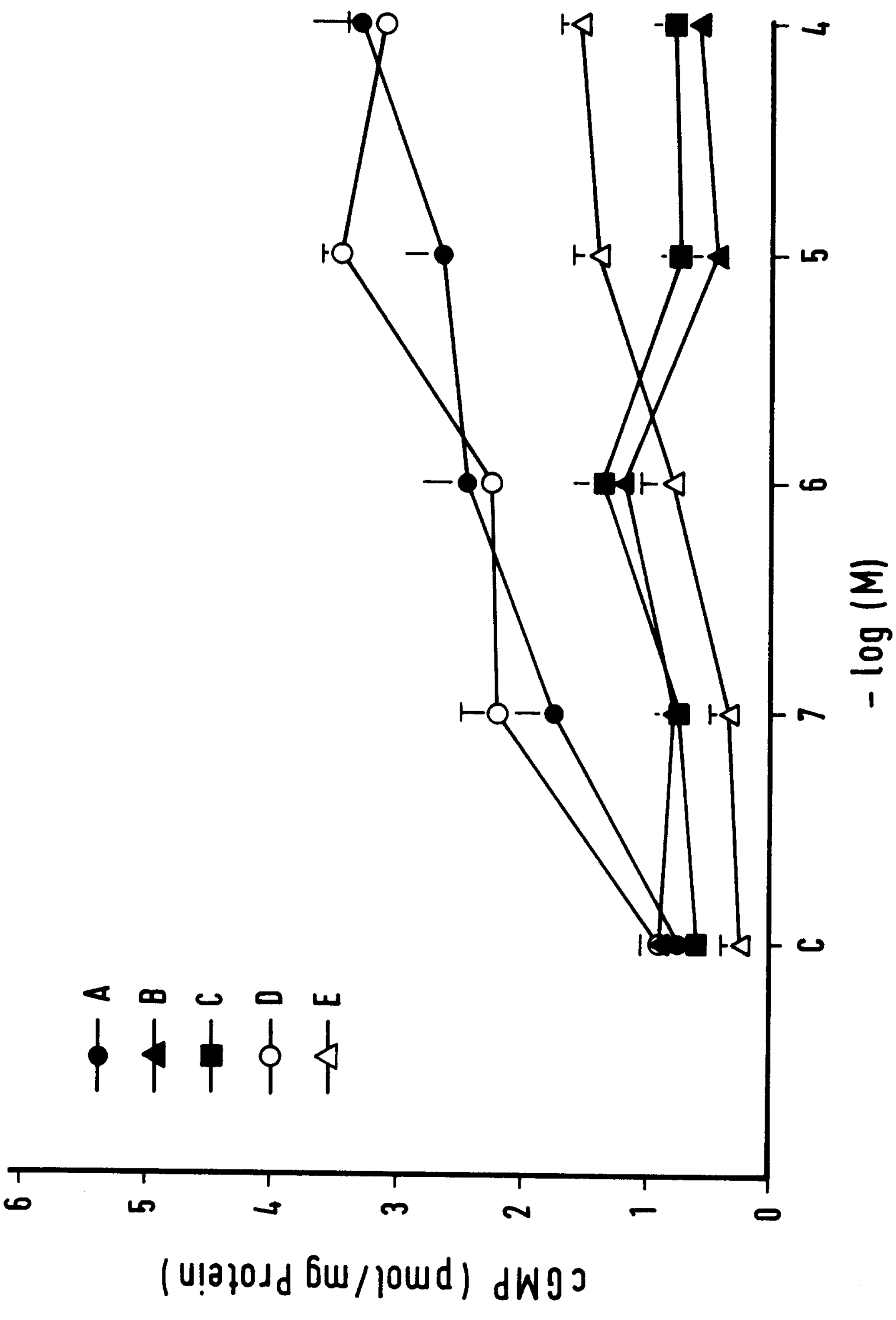

Fig. 2

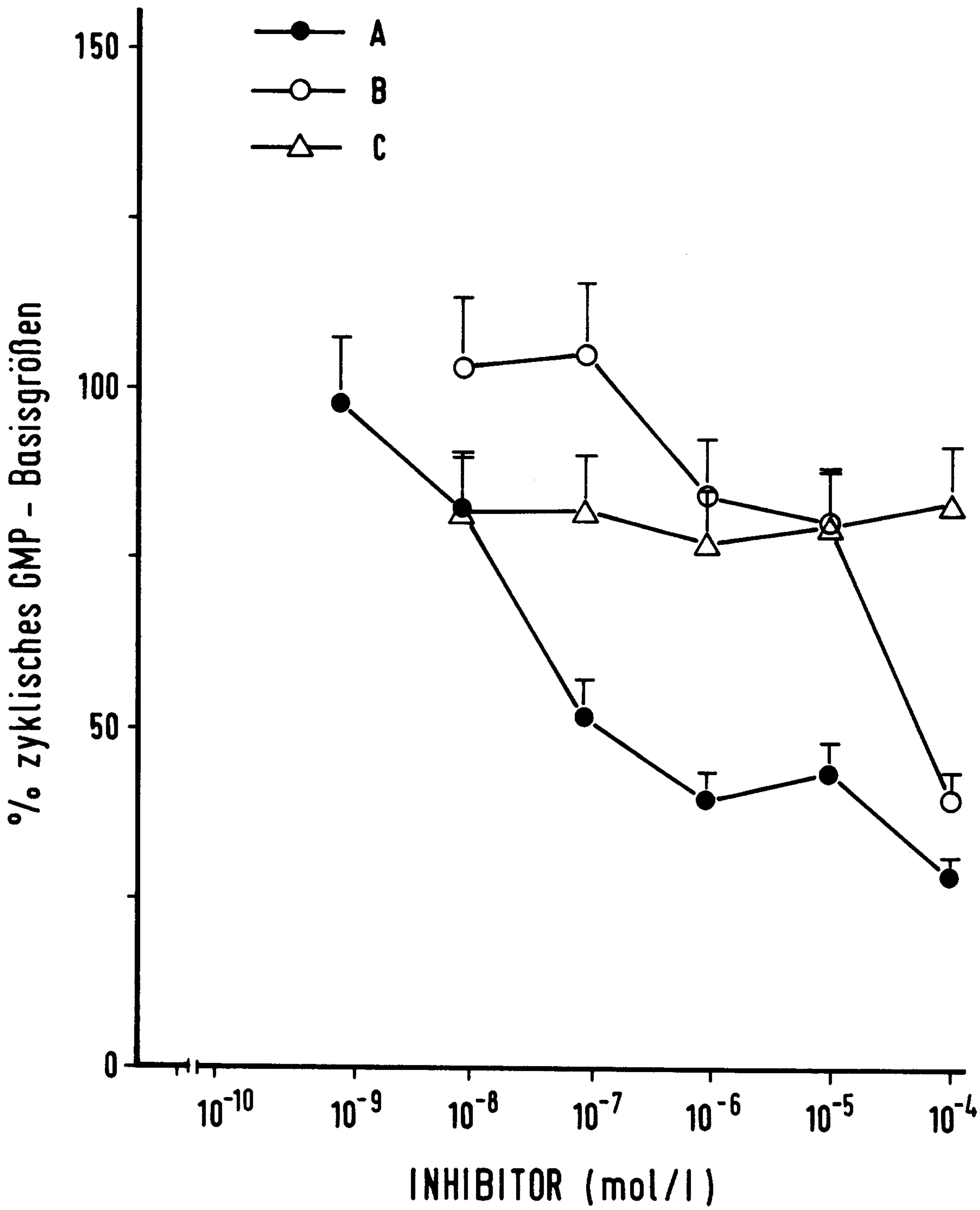
A
B
C
150
100
50
0
% zyklisches GMP - Basisgrößen
$10^{-10}$
$10^{-9}$
$10^{-8}$
$10^{-7}$
$10^{-6}$
$10^{-5}$
$10^{-4}$
INHIBITOR (mol/l)